# EUROPEAN PATENT SPECIFICATION

(11) **EP 1 268 798 B1**
(45) Date of publication and mention of the grant of the patent: **31.05.2006**
(21) Application number: 01962422.0
(22) Date of filing: 28.03.2001
(51) Int. Cl.: C12N 15/12, C12N 15/62, C12N 15/86, C07K 16/18, C07K 14/78, A61K 38/39, A01K 67/027

(54) **Anti-angiogenic properties of vascostatin and fragments or variants thereof**
Antiangiogene Eigenschaften von Vascostatin und Fragmenten und Varianten davon
Propriétés anti-angiogéniques de la proteine vascostatin et fragments et variants de celui-ci

(30) Priority: 29.03.2000 US 192871 P
(43) Date of publication of application: 02.01.2003
(73) Proprietor: Beth Israel Deaconess Medical Center, Inc., Boston, MA 02215 (US)
(72) Inventor: KALLURI, Raghuram, Boston, MA 02116 (US)
(74) Representative: White, Martin Paul
(86) International application number: PCT/US2001/040382
(87) International publication number: WO 2001/073025

(56) References cited:
- WO-A-95/22979
- MANN K ET AL: "AMINO ACID SEQUENCE OF MOUSE NIDOGEN A MULTIDOMAIN BASEMENT MEMBRANE PROTEIN WITH BINDING ACTIVITY FOR LAMININ COLLAGEN IV AND CELLS" EMBO (EUROPEAN MOLECULAR BIOLOGY ORGANIZATION) JOURNAL, vol. 8, no. 1, 1989, pages 65-72, XP002183115 ISSN: 0261-4189
- FOX J W ET AL: "RECOMBINANT NIDOGEN CONSISTS OF THREE GLOBULAR DOMAINS AND MEDIATES BINDING OF LAMININ TO COLLAGEN TYPE IV" EMBO (EUROPEAN MOLECULAR BIOLOGY ORGANIZATION) JOURNAL, vol. 10, no. 11, 1991, pages 3137-3146, XP002183116 ISSN: 0261-4189
- CAO, Y.: "Endogenous engiogenesis inhibitors: angiostatin, endostatin, and other proteolytic fragments." PROGRESS IN MOLECULAR AND SUBCELLULAR BIOLOGY, vol. 20, 1998, pages 161-176, XP001041400
- NICOSIA R F ET AL: "MODULATION OF ANGIOGENESIS IN VITRO BY LAMININ-ENTACTIN COMPLEX" DEVELOPMENTAL BIOLOGY, ACADEMIC PRESS, NEW YORK, NY, US, vol. 164, no. 1, July 1994 (1994-07), pages 197-206, XP000971040 ISSN: 0012-1606

## Description

### BACKGROUND OF THE INVENTION

Basement membranes are thin layers of specialized extracellular matrix that provide supporting structure on which epithelial and endothelial cells grow, and that surround muscle or fat (Paulsson, M., 1992, *Crit*. *Rev. Biochem. Mol. BioL* 27:93-127). Basement membranes are always associated with cells, and it has been well documented that basement membranes not only provide mechanical support, but also influence cellular behavior such as differentiation and proliferation. Vascular basement membranes are composed of macromolecules such as collagen, laminin, heparan sulfate proteoglycans, fibronectin and nidogen (also called entactin) (Timpl, R, 1996, *Curr. Opin. Cell. Biol.* 8:618-24).

Angiogenesis is the process of formation of new blood vessels from pre-existing ones (Madri, J.A. *et al.,* 1986, *J. Histochem. Cytochem.* 34:85-91; Follonan, J., 1972, *Ann. Surg.* 175:409-16). Angiogenesis is a complex process, and requires sprouting and migration of endothelial cells, proliferation of those cells, and their differentiation into tube-like structures and the production of a basement membrane matrix around the developing blood vessel. Additionally angiogenesis is a process critical for normal physiological events such as wound repair and endometrium remodeling (Folkman, J. *et al.,* 1995, *J. Biol. Chem.* 267:10931-34). It is now well documented that angiogenesis is required for metastasis and growth of solid tumors beyond a few mm³ in size (Folkman, J., 1972, *Ann. Surg.* 175:409-16; Folkman, J., 1995, *Nat. Med.* 1:27-31). Expansion of tumor mass occurs not only by perfusion of blood through the tumor, but also by paracrine stimulation of tumor cells by several growth factors and matrix proteins produced by the new capillary endothelium (Folkman, J., 1995, *Nat. Med.* 1:27-31). Recently, a number of angiogenesis inhibitors have been identified, namely angiostatin (O'Reilly, M.S. *et al.,* 1994, *Cell* 79:315-28), endostatin (O'Reilly, MS. *et al.,* 1997, *Cell* 88:277-85), restin (Ramchandran, R. *et al.,* 1999, *Biochem. Biophys. Res. Commun.* 255:735-9), Arrestin (Colorado, P.C. *et al,* 2000, *Cancer Res.* 60:2520-6), Canstatin (Kamphaus, G.D. *et al.,* 2000, *J. Biol Chem.* 275:1209-15) and Tumstatin (Maeshima, *Y. et al.,* 2000, *J. Biol. Chem.* 275:21340-8; Maeshinia, Y. *et al.,* 2000, *J. Biol. Chem.* 275:23745-50) and pigment epithelium-derived factor (PEDF) (Dawson, D.W. *et al.,* 1999, *Science* 285:245-8).

### SUMMARY OF THE INVENTION

The present invention relates to a protein of SEQ ID No:2, or a fragment comprising at least 25 continuous amino acids of said protein, or a variant of the protein having at least 70% sequence identity with SEQ ID No:2; wherein said protein, variant or fragment has anti-angiogenic activity.

The present invention includes a protein (designated herein as "Vascostatin") that consists of the C-terminal globular domain of nidogen-1. The nidogen-1 is preferably mammalian.

The Vascostatin may be about 19 to about 21 Kda in size, or about 20 Kda in size.

Preferred variants of the present invention have at least 80% or at least 90% sequence identity with the protein of SEQ ID No:2.

The protein, fragment, or variant may be in monomeric or a multimeric form (e.g. dimeric or trimeric).

Also within the scope of the present invention is a chimeric protein (also known as a fusion protein) comprising the protein, fragment or variant thereof.

The chimeric protein preferably comprises at least one protein molecule selected from the group consisting of: matin or fragments thereof, arrestin or fragments thereof, canstatin or fragments thereof, tumstatin or fragments thereof, endostatin or fragments thereof, angiostatin or fragments thereof, restin or fragments thereof, apomigren or fragments thereof, or other anti-angiogenic proteins or fragments thereof.

(Matin comprises a globular domain of laminin, preferably the G1 domain, and inhibits endothelial cell proliferation *in vivo.* Matin is described in international patent application number PCT/US01/09921, entitled "Anti-angiogenic and Antitumor Properties of Matin and Other Laminin Domains", by Raghuram Kalluri, as filed on March 28, 2001.)

The invention also features a polynucleotide encoding the protein, fragment, variant or chimeric protein of the present invention.

The polynucleotide may be operably linked to an expression control sequence.

A polynucleotide of the present invention can be used to provide probes that hybridize under conditions of moderate or high stringency thereto. The present invention includes a method comprising
(a) preparing one or more polynucleotide probes that hybridize under conditions of moderate stringency or high stringency to the polynucleotide;
(b) hybridizing said probe(s) to mammalian DNA; and
(c) isolating the DNA polynucleotide detected with the probe(s); wherein the nucleotide sequence of the isolated polynucleotide corresponds to the nucleotide sequence of the polynucleotide of the present invention.

The probes may for example be SEQ ID NO:3 or SEQ ID NO:4. The isolated polynucleotide may, for example, be a subsequence of SEQ ID NO:1.

A polynucleotide of the present invention can be used (with or without operable linkage to an expression control sequence) to transform a host cell. The host cell may be selected from the group comprising bacterial, yeast, mammalian, insect or plant cells.

The present invention therefore includes a host cell, such as bacterial, yeast, mammalian, insect or plant cell, transformed with a polynucleotide of the present invention, with the proviso that the host cell is not part of a human embryo.

The polynucleotide or host cell can be used in medicine.

The present invention includes the use of the polynucleotide in the preparation of a therapeutic mammalian cell for treating a mammal having a disorder as previously described; wherein the preparation involves treating a mammalian cell *in vitro* to insert therein the polynucleotide; and wherein said cell is not part of a human embryo.

The mammalian cell may be chosen from the group consisting of: blood cells, TIL cells, bone marrow cells, vascular cells, tumor cells, liver cells, muscle cells, fibroblast cells. The polynucleotide may be inserted into the cells by a viral vector.

The cell may allow transient expression.

Mammalian host cells may be used in a process for providing a mammal with an anti-angiogenic protein, where the mammalian cells express *in vivo* within the mammal a therapeutically effective amount of the anti-angiogenic protein in an amount sufficient to inhibit angiogenic activity in the mammal.

A host cell of the present invention can be used to produce a protein, fragment, variant, monomer, multimer, or chimeric protein of the present invention by the steps of
a) growing the host cell in culture and
b) purifying a protein, fragment, variant, monomer, multimer, or chimeric protein encoded by the polynucleotide within the host cell from the culture.

The present invention also includes a pharmaceutical or contraceptive composition comprising a protein, fragment, variant, monomer, multimer, or chimeric protein of the present invention.

The composition may comprise a pharmaceutically compatible carrier.

It may comprise at least one protein molecule selected from the group consisting of: matin or fragments thereof, arrestin or fragments thereof, canstatin or fragments thereof, tumstatin or fragments thereof, endostatin or fragments thereof, angiostatin or fragments thereof, restin or fragments thereof, apomigren or fragments thereof, or other anti-angiogenic proteins, or fragments thereof.

The present invention further includes the fragment, variant, monomer, multimer, chimeric protein, and composition of the present invention; for use in medicine.

The fragment, variant, monomer, multimer, chimeric protein, and composition can be used to contact mammalian tissue and can therefore be used in the treatment of mammals.

The protein, fragment, variant, monomer, multimer, chimeric protein, or composition can be used in the preparation of a medicament for treating a disorder involving angiogenesis.

The disorder may be cancer. It may involve tumor growth.

The present invention includes the use of a protein, fragment, variant, monomer, multimer, chimeric protein or composition of the present invention in the preparation of a medicament for treating a disorder involving endothelial cell proliferation.

The present invention further includes the use of the protein, fragment, variant, monomer, multimer, chimeric protein or composition in the preparation of a medicament for treating a disorder selected from the group comprising angiogenesis-dependent cancers, benign tumors, rheumatoid arthritis, diabetic retinopathy, fibrosis, psoriasis, ocular angiogenesis diseases, Osler-Webber Syndrome, myocardial angiogenesis, plaque neovascularization, telangiectasia, hemopheliac joints, angiofibroma, wound granulation, intestinal adhesions, atherosclerosis, scleroderma, hypertrophic scars, cat scratch disease, *Helicobacter pylori* ulcers, dialysis graft vascular access stenosis and obesity.

The protein, fragment, variant, monomer, multimer, chimeric protein or composition may be combined with a radiotherapeutic, chemotherapeutic or immunotherapeutic agent; for simultaneous, sequential or separate use in anti-cancer therapy.

In addition to the various aspects discussed above, the present invention also includes the protein, fragment, variant, monomer, multimer, chimeric protein, polynucleotide or host cell in isolated form.

### BRIEF DESCRIPTION OF THE DRAWINGS

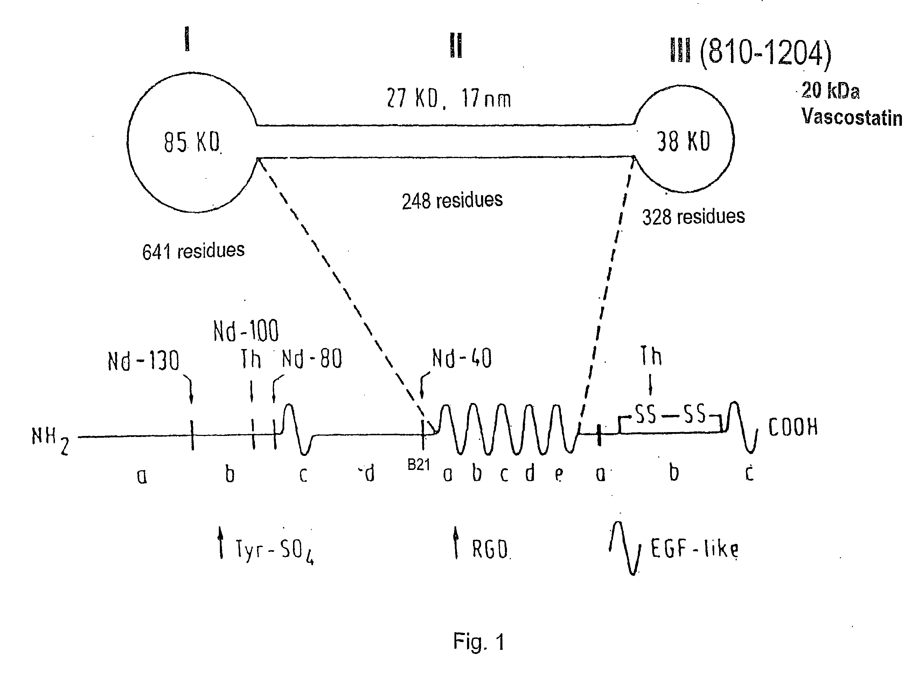

Fig. 1 is a schematic diagram of Nidogen-1, with the C-terminal globular domain (*i.e.*, Vascostatin) indicated.

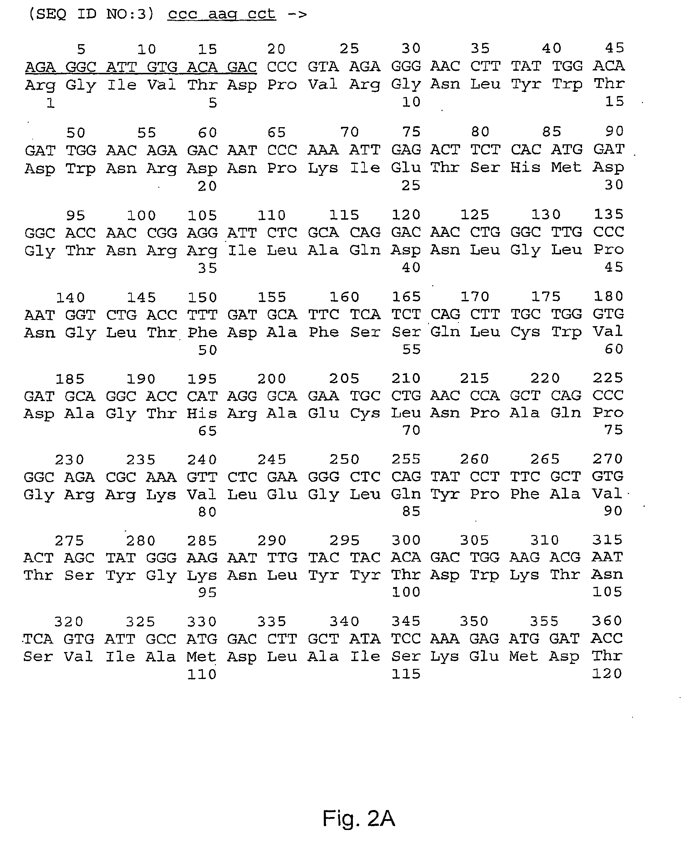

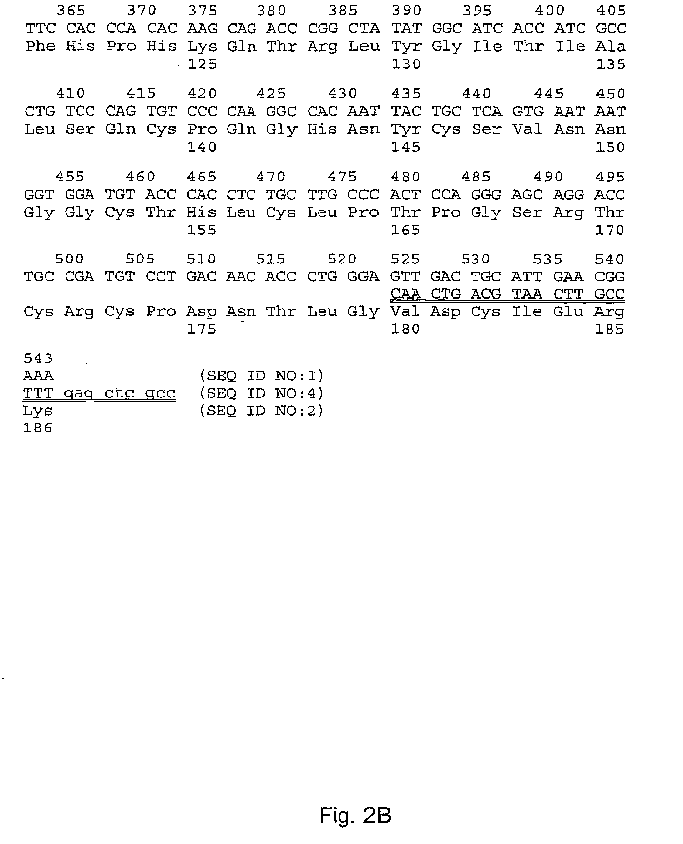

Figs. 2A and 2B are a diagram depicting the nucleotide (SEQ ID NO:1) and amino acid (SEQ ID NO:2) sequences of Vascostatin, the C-terminal globular domain of nidogen-1 (GenBank Acc. No. X14480). The locations of the pET22b(+) forward (5'-CCC-AAG-CTT-AGA-GGC-ATT-GTG-ACA-GAC-3 ; SEQ ID NO:3) and reverse (5'-CCG-CTC-GAG-TTT-CCG-TTC-AAT-GCA GTC-AAC-3'; SEQ ID NO:4) primers are indicated by single and double underlining, respectively. Nucleotides in lower case are not derived from nidogen-1, but represent the portion of the primer derived from vector sequence.

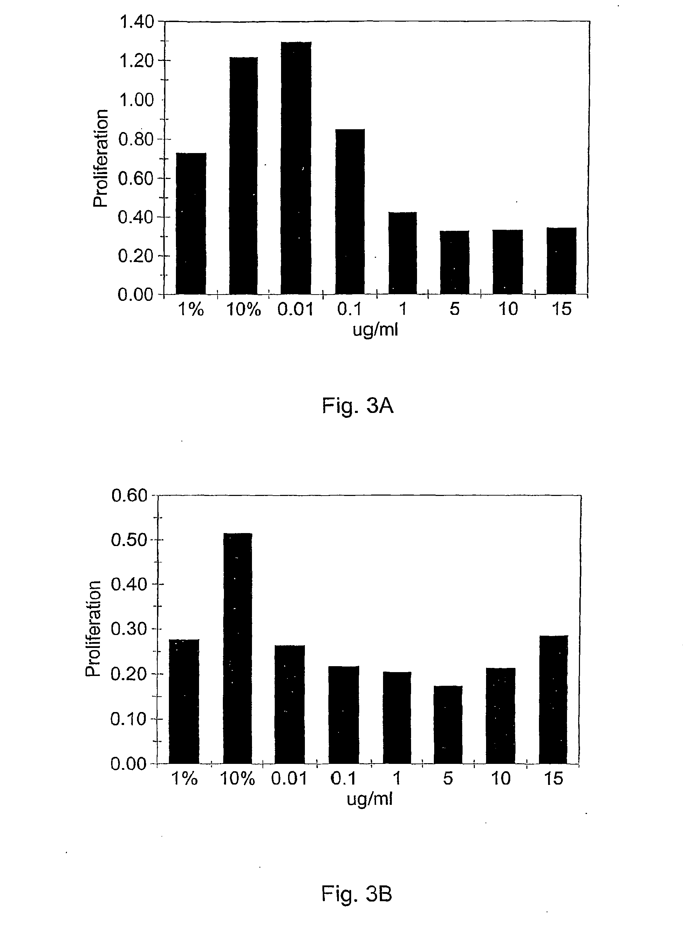

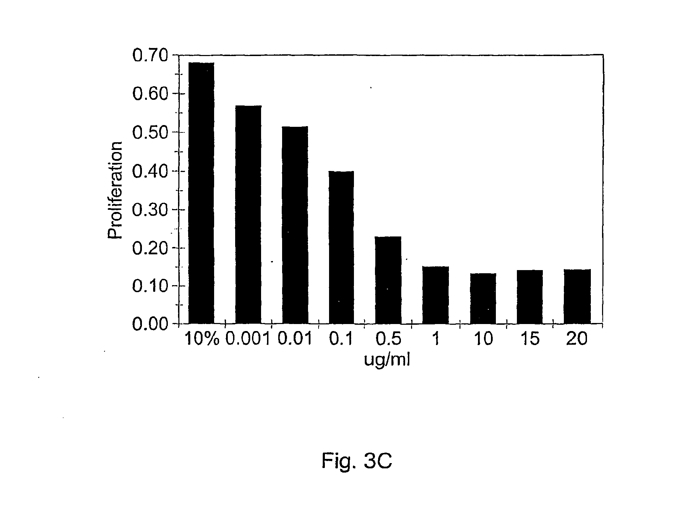

Figs. 3A, 3B and 3C are three histograms showing the effect of Vascostatin on the proliferation of endothelial (C-PAE) cells. Absorbance at OD₆₅₅ is shown on the y-axis. The x-axis shows treatments varying amounts of FCS or Vascostatin. Fig. 3A shows treatments of 1% FCS, 10% FCS, and 0.01, 0.1, 1.0, 5.0, 10.0 and 15.0 µg/ml Vascostatin, while Fig. 3B shows treatments of 0.1% FCS, 10% FCS, and 0.01, 0.1, 1.0, 5.0, 10.0 and 15.0 µg/ml Vascostatin, and Fig. 3C shows treatments of 10% FBS, and 0.001, 0.01, 0.1, 0.5, 1.0, 10.0, 15.0 and 20.0 µg/ml Vascostatin.

### DETAILED DESCRIPTION OF THE INVENTION

A wide variety of diseases are the result of undesirable angiogenesis. Put another way, many diseases and undesirable conditions could be prevented or alleviated if it were possible to stop the growth and extension of capillary blood vessels under some conditions, at certain times, or in particular tissues.

The formation of new capillaries from pre-existing vessels, angiogenesis, is essential for the process of tumor growth and metastasis (Folkman, J. *et al.,* 1992, *J*. *Biol. Chem.* 267:10931-4; Folkman, J., 1995, *Nat. Med.* 1:27-31; Hanahan, *D. et al.,* 1996, *Cell* 86:353-64). Human and animal tumors are not vascularized at the beginning, however for a tumor to grow beyond few mm³, it might vascularize (Follanan, J., 1995, *Nat. Med.* 1:27-31; Hanahan, D*. et al.,* 1996, *Cell* 86:353-64). The switch to an angiogenic phenotype requires both upregulation of angiogenic stimulators and downregulation of angiogenesis inhibitors (Folkman, J., 1995, *Nat. Med.* 1:27-31). Vascular endothelial growth factor (VEGF) and basic fibroblast growth factor (bFGF) are the most commonly expressed angiogenic factors in tumors. Vascularized tumors may overexpress one or more of these angiogenic factors which can synergistically promote tumor growth. Inhibition of a single angiogenic factor such as VEGF with a receptor antagonist may not be enough to arrest tumor growth. A number of angiogenesis inhibitors have been recently identified, and certain factors such as IFN-α, platelet-factor-4 (Maione, T.E. *et al., 1990, Science* 247:77-9) and PEX (Brooks, P.C. *et al.,* 1998, *Cell* 92:391-400) are not endogenously associated with tumor cells, whereas angiostatin (O'Reilly, M.S. *et al.,* 1994, *Cell* 79:315-28) and endostatin (O'Reilly, M.S. *et al.,* 1997, *Cell* 88:277-85) are tumor associated angiogenesis inhibitors generated by tumor tissue itself. Although treatment of tumor growth and metastasis with these endogenous angiogenesis inhibitors is very effective and an attractive idea, some potential problems associated with anti-angiogenic therapies must be considered. Delayed toxicity induced by chronic anti-angiogenic therapy as well as the possibility of impaired wound healing and reproductive angiogenesis occurring during treatment are to be considered seriously.

In the present invention, a protein, and fragments, analogs, derivatives, homologs and mutants thereof with anti-angiogenic properties are described, along with methods of use of this protein, analogs, derivatives, homologs and mutants to inhibit angiogenesis-mediated proliferative diseases. The protein comprises the C-terminal globular domain of nidogen-1, and is called "Vascostatin." This protein is about 20 kDa, and inhibits endothelial cell proliferation.

Nidogen, also known as entactin, is a component of basement membranes, and is often found occurring with laminin. The nidogen protein has a roughly dumbbell shape, with domains I and III forming the larger and smaller of the two globules, respectively, and domain II forming a rod-like structure connecting the two. Nidogen was initially isolated from EHS (Engelbreth-Holm-Swarm) tumors (Timpl, R *et al.,* 1983, *Eur. J. Biochem.* 137:455-465), and entactin from mouse endodermal cell line M1536-B3 (Carlin, B *et al.,* 1981, *J. Biol. Chem.* 256:5209-5214). The two molecules were later found to be identical. Nidogen is also sometimes referred to as "laminin C chain" (Paulsson, M., 1992, *Crit. Rev. Biochem. Mol. Biol.* 27:93-127) because it is expressed and complexed noncovalently together with laminin in normal basement membrane.

Domain III of mouse and human nidogen share about 85% homology at the amino acid level, and show conservation of structure (Olsen, *D.R. et al.,* 1989, *Am. J. Hum. Genet.* 44:876-885; Nagayashi, T. *et al.,* 1989, *DNA* 8:581-594). Nidogen and laminin are often found occurring together, and domain III is responsible for mediating this interaction (Mann, K. *et al.,* 1988, *Eur. J. Biochem.* 178:71-81).

Vascostatin can be obtained from several sources. Nidogen and nidogen-1 from human and mouse, for instance, generally possess 90% or greater sequence identity with SEQ ID NO:2. Such sequences include, but are not limited to, GenBank accessions NP_035047 (nidogen [Mus musculus]), P10493 (NIDOGEN PRECURSOR (ENTACTIN)), MMMSND (nidogen precursor - mouse), CAA32408 (enactin precursor (AA -28 to 1217) [Mus musculus]), 1504282A (entactin), MMHUND (nodogen procursor - human), XP_002042 (nidogen (enactin) [Homo sapiens]), NP_002499 (nidogen (enactin); Nidogen; nidogen (entactin) [Homo sapiens]), P14543 (NIDOGEN PRECURSOR (ENTACTIN)), AAA59932 (nidogen [Homo sapiens]), CAA57709 (nidogen [Homo sapiens]) and AAA57261 (nidogen [Homo sapiens]). Interestingly, these same sequences also possess a second region of identity homology with SEQ ID NO:2, generally exhibiting 27-28% identity with SEQ ID NO:2.

Nidogens from other animals, *e.g., Gallus gallus* (AAF44680 (nidogen 1; entactin [Gallus gallus])), have lower sequence identities with SEQ ID NO:2, *e.g.,* 75%, but still possess the second region of weaker identity. It therefore seems likely that such other sequences possess properties, e.g., anti-angiogenic properties, similar to those of the mammalian sequences.

Nidogen-2 sequences (e.g., NP_032721 (nidogen 2 [Mus musculus]); 088322 (NIDOGEN-2 PRECURSOR (NID-2) (ENTACTIN-2)); BAA32609 (entactin-2 [Mus musculus]); Q14112 (NIDOGEN-2 PRECURSOR (NID-2) (OSTEONIDOGEN)); CAA11418 (nidogen-2 [Homo sapiens]); NP_031387 (nidogen 2; nidogen 2 (osteonidogen) [Homo sapiens]); G00043 (osteonidogen - human); BAA24112 (osteonidogen [Homo sapiens]); BAA13087 (osteonidogen [Homo sapiens])) possess identities of about 50% or greater with SEQ ID NO:2.

These sequences, and their identities with SEQ ID NO:2, are shown in Table 1, below. "Vascostatin", as defined herein, is therefore intended to include anti-angiogenic proteins and fragments from these and other such sources.

**Table 1. GenBank Accessions having sequence identity with SEQ ID NO:2.**

| | | Regions of identity with SEQ ID NO:2 | | |
|---|---|---|---|---|
| GenBank Acc. No. | Protein (source organism) | Residues in Acc. | Residues in SEQ ID NO:2 | % Ident. |
| NP_035047 | nidogen (mouse) | 1065-1245 | 1-181 | 100 |
| | | 980-1085 | 2-106 | 27 |
| P10493 | nidogen (mouse) | 1065-1245 | 1-181 | 100 |
| | | 980-1085 | 2-106 | 27 |
| MMMSND | nidogen (mouse) | 1065-1245 | 1-181 | 100 |
| | | 980-1085 | 2-106 | 27 |
| CAA32408 | nidogen (mouse) | 1065-1245 | 1-181 | 100 |
| | | 980-1085 | 2-106 | 27 |
| 1504282A | nidogen (mouse) | 1065-1245 | 1-181 | 100 |
| | | 980-1085 | 2-106 | 27 |
| MMHUND | nidogen (human) | 1067-1247 | 1-181 | 90 |
| | | 982-1087 | 2-106 | 28 |
| XP_002042 | nidogen (human) | 1097-1277 | 1-181 | 90 |
| | | 1012-1117 | 2-106 | 28 |
| NP_002499 | nidogen (human) | 1067-1247 | 1-181 | 90 |
| | | 982-1087 | 2-106 | 28 |
| P14543 | nidogen (human) | 1067-1247 | 1-181 | 90 |
| | | 982-1087 | 2-106 | 28 |
| AAA59932 | nidogen (human) | 1067-1247 | 1-181 | 90 |
| | | 982-1087 | 2-106 | 28 |
| CAA57709 | nidogen (human) | 1066-1246 | 1-181 | 90 |
| | | 981-1086 | 2-106 | 28 |
| AAA57261 | nidogen (human) | 401-581 | 1-181 | 90 |
| | | 316-421 | 2-106 | 28 |
| AAF44680 | nidogen-1 (goose) | 294-473 | 1-180 | 75 |
| | | 209-310 | 2-102 | 29 |
| NP_032721 | nidogen-2 (mouse) | 1260-1401 | 1-142 | 52 |
| 088322 | nidogen-2 (mouse) | 1260-1401 | 1-142 | 52 |
| BAA32609 | nidogen-2 (mouse) | 1260-1401 | 1-142 | 52 |
| Q14112 | nidogen-2 (human) | 1232-1373 | 1-142 | 51 |
| CAA11418 | nidogen-2 (human) | 1232-1373 | 1-142 | 51 |
| NP_031387 | nidogen-2 (human) | 1233-1374 | 1-142 | 51 |
| G00043 | nidogen-2 (human) | 1233-1374 | 1-142 | 51 |
| BAA13087 | nidogen-2 (human) | 1233-1374 | 1-142 | 51 |
| BAA24112 | nidogen-2 (human) | 1233-1374 | 1-142 | 51 |

Polynucleotides encoding Vascostatin can also be obtained from a variety of sources. For instance, other mouse nidogen polynucleotides (*e*.*g*., NM_010917 (Mus musculus nidogen 1 (Nid1), mRNA); X14194 (Mouse mRNA for entactin (MOUSE);, mRNA sequence)) generally possess greater than 90% sequence identity with SEQ ID NO:1, and human nidogen full-length polynucleotides (e.g., M27445 (Homo sapiens nidogen (NID) mRNA, 3' end); XM_002042 (Homo sapiens nidogen (enactin) (NID), mRNA); NM_002508 (Homo sapiens nidogen (enactin) (NID), mRNA); M30269 (NIDOGEN PRECURSOR (HUMAN);, mRNA sequence)) have 85% or greater sequence identity.

Mouse and human exon sequences (e.g., X83090 (M.musculus nid gene (exon 16)); X83091 (M.musculus nid gene (exon 17)); X83092 (M.musculus nid gene (exon 18)); X83093 (M.musculus nid gene (exons 19 & 20)); X84834 (H.sapiens nidogen gene (exon 17)); X84835 (H.sapiens nidogen gene (exon 18)); X84836 (H.sapiens nidogen gene (exon 19)); X84837 (H.sapiens nidogen gene (exon 20))) are generally shorter than SEQ ID NO:1, but have similar levels of sequence identity, e.g., 90% or greater, and 85% or greater, respectively. As was the case with the protein sequences, non-mammalian nidogen (e.g., AF239837 (Gallus gallus nidogen 1 mRNA, partial cds)) was found to have a lower level of sequence identity, e.g., around 70-75%.

These sequences, and their identities with SEQ ID NO:1, are shown in Table 2, below. A polynucleotide encoding Vascostatin is therefore intended to include polynucleotides from these and other such sources, where the polynucleotide encodes an anti-angiogenic protein or anti-angiogenic fragment.

**Table 2. GenBank Accessions having sequence identity with SEQ ID NO:1.**

| | | Regions of identity with SEQ ID NO:1 | | |
|---|---|---|---|---|
| GenBank Acc. No. | Protein (source organism) | Bases in Acc. | Bases in SEQ ID NO:1 | % Ident. |
| NM_010917 | nidogen-1 (mouse) | 3204-3756 | 1-543 | 90 |
| X14194 | nidogen (mouse) | 3204-3756 | 1-543 | 99 |
| X83090 | nidogen (mouse) | 157-186 | 1-30 | 100 |
| X83091 | nidogen (mouse) | 14-173 | 29-188 | 100 |
| X83092 | nidogen (mouse) | 14-141 | 185-312 | 100 |
| X83093 | nidogen (mouse) | 524-644 | 423-543 | 100 |
| | | 13-126 | 312-425 | 100 |
| M27445 | nidogen (human) | 1201-1743 | 1-543 | 86 |
| XM_002042 | nidogen (human) | 3289-3831 | 1-543 | 86 |
| NM_002508 | nidogen (human) | 3289-3831 | 1-543 | 86 |
| M30269 | nidogen (human) | 3289-3831 | 1-543 | 86 |
| X84834 | nidogen (human) | 6-165 | 29-188 | 86 |
| X84835 | nidogen (human) | 4-129 | 185-310 | 88 |
| X84836 | nidogen (human) | 5-125 | 334-425 | 88 |
| X84837 | nidogen (human) | 29-120 | 423-543 | 85 |
| AF239837 | nidogen-1 (goose) | 921-1355 | 40-474 | 73 |

As disclosed herein, Vascostatin can be can be produced in *E. coli* using a bacterial expression plasmid, such as pET22b, which is capable of periplasmic transport, thus resulting in soluble protein. Vascostatin can also be produced in other cells, for instance, it can be produced as a secreted soluble protein in 293 kidney cells using the pcDNA 3.1 eukaryotic vector.

*E. coli*-produced Vascostatin inhibits endothelial cell proliferation of endothelial cells in a dose-dependent manner.

Specific inhibition of endothelial cell proliferation and migration by Vascostatin demonstrates its anti-angiogenic activity, and that it may function via a cell surface protein/receptor. Integrins are potential candidate molecules based on their extracellular matrix binding capacity and ability to modulate cell behavior such as migration and proliferation. In particular, aᵥb₃ integrin is a possible receptor, due to its induction during angiogenesis, and its promiscuous binding capacity. Angiogenesis also depends on specific endothelial cell adhesive events mediated by integrin aᵥb₃ (Brooks, P.C. *et al.*, 1994, *Cell* 79:1157-64). Vascostatin may disrupt the interaction of proliferating endothelial cells to the matrix component, and thus drive endothelial cells to undergo apoptosis (Re, F. *et al.,* 1994, *J. Cell. Biol.* 127:537-46). Matrix metalloproteinases (MMP's) have been implicated as key enzymes that regulate the formation of new blood vessels in tumors (Ray, J.M. *et al.,* 1994, *Eur. Respir. J.* 7:2062-72). Recently, it was demonstrated that an inhibitor of MMP-2 (PEX) can suppress tumor growth by inhibiting angiogenesis (Brooks, P.C. *et al.,* 1998, *Cell* 92:391-400). Vascostatin may function through inhibiting the activity of MMPs.

As used herein, the term "angiogenesis" means the generation of new blood vessels into a tissue or organ, and involves endothelial cell proliferation. Under normal physiological conditions, humans or animals undergo angiogenesis only in very specific restricted situations. For example, angiogenesis is normally observed in wound healing, fetal and embryonal development, and formation of the corpus luteum, endometrium and placenta. The term "endothelium" means a thin layer of flat epithelial cells that lines serous cavities, lymph vessels, and blood vessels. "Anti-angiogenic activity" therefore refers to the capability of a composition to inhibit the growth of blood vessels. The growth of blood vessels is a complex series of events, and includes localized breakdown of the basement membrane lying under the individual endothelial cells, proliferation of those cells, migration of the cells to the location of the future blood vessel, reorganization of the cells to form a new vessel membrane, cessation of endothelial cell proliferation, and, incorporation of pericytes and other cells that support the new blood vessel wall. "Anti-angiogenic activity" as used herein therefore includes interruption of any or all of these stages, with the end result that formation of new blood vessels is inhibited.

Anti-angiogenic activity may include endothelial inhibiting activity, which refers to the capability of a composition to inhibit angiogenesis in general and, for example, to inhibit the growth or migration of bovine capillary endothelial cells in culture in the presence of fibroblast growth factor, angiogenesis-associated factors, or other known growth factors. A "growth factor" is a composition that stimulates the growth, reproduction, or synthetic activity of cells. An "angiogenesis-associated factor" is a factor which either inhibits or promotes angiogenesis. An example of an angiogenesis-associated factor is an angiogenic growth factor, such as basic fibroblastic growth factor (bFGF), which is an angiogenesis promoter. Another example of an angiogenesis-associated factor is an angiogenesis inhibiting factor such as *e.g.*, angiostatin (see, e.g., U.S. Pat. No. 5,801,012, U.S. Pat. No. 5,837,682, U.S. Pat. No. 5,733,876, U.S. Pat. No. 5,776,704, U.S. Pat. No. 5,639,725, U.S. Pat. No. 5,792,845, WO 96/35774, WO 95/29242, WO 96/41194, WO 97/23500) or endostatin (see, *e.g.*, U.S. Pat. No. 5,854,205; U.S. Pat. No. 6,174,861; WO 97/15666).

By "substantially the same biological activity" or "substantially the same or superior biological activity" is meant that a composition has anti-angiogenic activity, and behaves similarly as does Vascostatin, as determined in standard assays. "Standard assays" include, but are not limited to, those protocols used in the molecular biological arts to assess anti-angiogenic activity, cell cycle arrest, and apoptosis. Such assays include, but are not limited to, assays of endothelial cell proliferation, endothelial cell migration, cell cycle analysis, and endothelial cell tube formation, detection of apoptosis, e.g., by apoptotic cell morphology or Annexin V-FITC assay, chorioallantoic membrane (CAM) assay, and inhibition of renal cancer tumor growth in nude mice. Such assays are provided in the Examples below, and also in U.S.S.N. 09/335,224, "Anti-Angiogenic Proteins and Methods of Use thereof," filed June 17, 1999, by Raghuram Kalluri, and in U.S.S.N. 09/479,118, "Anti-Angiogenic Proteins and Receptors and Methods of Use thereof," by Raghuram Kalluri, filed January 7, 2000.

"Vascostatin" is intended to include fragments, mutants, homologs, analogs, and allelic variants of the amino acid sequence of the Vascostatin sequence, as well as Vascostatin from other nidogens, nidogens from other mammals, and fragments, mutants, homologs, analogs and allelic variants of the Vascostatin amino acid sequence.

It is to be understood that the present invention is contemplated to include any derivatives of Vascostatin within the scope of the claims that have endothelial inhibitory activity (*e.g*., the capability of a composition to inhibit angiogenesis in general and, for example, to inhibit the growth or migration of bovine capillary endothelial cells in culture in the presence of fibroblast growth factor, angiogenesis-associated factors, or other known growth factors). The present invention includes the entire Vascostatin protein, derivatives of this protein and biologically-active fragments of this protein. This includes proteins with Vascostatin activity that have amino acid substitutions or have sugars or other molecules attached to amino acid functional groups.

The invention also describes fragments, mutants homologs and analogs of Vascostatin that are within the scope of the claims. A "fragment" of a protein is defined herein as any amino acid sequence shorter than that protein, comprising at least 25 consecutive amino acids of the full polypeptide. Such a fragment may alternatively comprise 26, 27, 28, 29, 30, 31, 32, 33, 34, 35, 36, 37, 38, 39, 40, 41, 42, 43, 44, 45, 46, 47, 48, 49 or 50 consecutive amino acids of the full polypeptide. The fragment may comprise 51, 52, 53, 54, 55, 56, 57, 58, 59, 60, 61, 62, 63, 64, 65, 66, 67, 68, 69, 70, 71, 72, 73, 74 or 75 consecutive amino acids of the full polypeptide. Such molecules may or may not also comprise additional amino acids derived from the process of cloning, *e.g.*, amino acid residues or amino acid sequences corresponding to full or partial linker sequences. To be encompassed by the present invention, such molecules, with or without such additional amino acid residues, must have substantially the same biological activity as the reference polypeptide.

Where the full-length molecule possesses more than one activity, e.g., it may be possible to "split" the activities by splitting the full-length protein into several fragments, e.g., the full-length protein can be split into two fragments, one of which may possess one activity, while the other possesses another activity. The two fragments may or may not overlap, and the two activities may or may not be apparent in the full-length molecule. For instance, the full-length molecule may possess activity "A", and two fragments whereof may possess activities "A₁" and "A₂", respectively, or they may possess activities "B" and "C". Therefore, when it is stated that a fragment or mutant "must have substantially the same biological activity as the reference polypeptide", it is intended that in situations where one or more biological activities are split, the "referenre polypeptide" is that subsequence of the overall molecule that corresponds to the fragment or mutant. That is, the fragment or mutant must have the substantially the same biological activity as that portion of the overall molecule to which they correspond.

By "mutant" of Vascostatin is meant a polypeptide that includes any change in the amino acid sequence relative to the amino acid sequence of the equivalent reference Vascostatin polypeptide. Such changes can arise either spontaneously or by manipulations by man, by chemical energy (e.g., X-ray), or by other forms of chemical mutagenesis, or by genetic engineering, or as a result of mating or other forms of exchange of genetic information. Mutations include, e.g., base changes, deletions, insertions, inversions, translocations, or duplications. Mutant forms of Vascostatin may display either increased or decreased anti-angiogenic activity relative to the equivalent reference Vascostatin polynucleotide, and such mutants may or may not also comprise additional amino acids derived from the process of cloning, e.g., amino acid residues or amino acid sequences corresponding to full or partial linker sequences. Mutants/fragments of the anti-angiogenic proteins of the present invention can also be generated by PCR cloning, or by *Pseudomonas* elastase digestion, as described by Mariyama, M. *et al.* (1992, *J. Biol. Chem.* 267:1253-1258).

By "analog" of Vascostatin is meant a non-natural molecule substantially similar to either the entire Vascostatin molecule or a fragment or allelic variant thereof, and having substantially the same or superior biological activity. Such analogs are intended to include derivatives *(e.g.,* chemical derivatives, as defined above) of the biologically active Vascostatin, as well as its fragments, mutants, homologs, and allelic variants, which derivatives exhibit a qualitatively similar agonist or antagonist effect to that of the unmodified Vascostatin polypeptide, fragment, mutant, homolog, or allelic variant.

By "allele" of Vascostatin is meant a polypeptide sequence containing a naturally-occurring sequence variation relative to the polypeptide sequence of the reference Vascostatin polypeptide. By "allele" of a polynucleotide encoding the Vascostatin polypeptide is meant a polynucleotide containing a sequence variation relative to the reference polynucleotide sequence encoding the reference Vascostatin polypeptide, where the allele of the polynucleotide encoding the Vascostatin polypeptide encodes an allelic form of the Vascostatin polypeptide.

It is possible that a given polypeptide may be either a fragment, a mutant, an analog, or allelic variant of Vascostatin, or it may be two or more of those things, e.g., a polypeptide may be both an analog and a mutant of the Vascostatin polypeptide. For example, a shortened version of the Vascostatin molecule (e.g., a fragment of Vascostatin) may be created in the laboratory. If that fragment is then mutated through means known in the art, a molecule is created that is both a fragment and a mutant of Vascostatin. In another example, a mutant may be created, which is later discovered to exist as an allelic form of Vascostatin in some mammalian individuals. Such a mutant Vascostatin molecule would therefore be both a mutant and an allelic variant. Such combinations of fragments, mutants, allelic variants, and analogs are intended to be encompassed in the present invention.

Encompassed by the present invention are proteins that have substantially the same amino acid sequence as Vascostatin, or polynucleotides that have substantially the same nucleic acid sequence as the polynucleotides encoding Vascostatin. "Substantially the same sequence" means a nucleic acid or polypeptide that exhibits at least about 70 % sequence identity with a reference sequence, e.g., another nucleic acid or polypeptide, typically at least about 80% sequence identity with the reference sequence, preferably at least about 90% sequence identity, more preferably at least about 95% identity, and most preferably at least about 97% sequence identity with the reference sequence. The length of comparison for sequences will generally be at least 75 nucleotide bases or 25 amino acids, more preferably at least 150 nucleotide bases or 50 amino acids, and most preferably 243-264 nucleotide bases or 81-88 amino acids. "Polypeptide" as used herein indicates a molecular chain of amino acids and does not refer to a specific length of the product. Thus, peptides, oligopeptides and proteins are included within the definition of polypeptide. This term is also intended to include polypeptide that have been subjected to post-expression modifications such as, for example, glycosylations, acetylations, phosphorylations and the like.

"Sequence identity," as used herein, refers to the subunit sequence similarity between two polymeric molecules, e.g., two polynucleotides or two polypeptides. When a subunit position in both of the two molecules is occupied by the same monomeric subunit, e.g., if a position in each of two peptides is occupied by serine, then they are identical at that position. The identity between two sequences is a direct function of the number of matching or identical positions, *e.g.,* if half *(e.g., 5* positions in a polymer 10 subunits in length) of the positions in two peptide or compound sequences are identical, then the two sequences are 50% identical; if 90% of the positions, e.g., 9 of 10 are matched, the two sequences share 90% sequence identity. By way of example, the amino acid sequences R₂R₅R₇R₁₀R₆R₃ and R₉R₈R₁R₁₀R₆R₃ have 3 of 6 positions in common, and therefore share 50% sequence identity, while the sequences R₂R₅R₇R₁₀R₆R₃ and R₈R₁R₁₀R₆R₃ have 3 of 5 positions in common, and therefore share 60% sequence identity. The identity between two sequences is a direct function of the number of matching or identical positions. Thus, if a portion of the reference sequence is deleted in a particular peptide, that deleted section is not counted for purposes of calculating sequence identity, *e.g.*, R₂R₅R₇R₁₀R₆R₃ and R₂R₅R₇R₁₀R₃ have 5 out of 6 positions in common, and therefore share 83.3% sequence identity.

Identity is often measured using sequence analysis software e.g., BLASTN or BLASTP (available at http://www.ncbi.nlm.nih.gov/BLAST/). The default parameters for comparing two sequences (e.g., "Blast"-ing two sequences against each other, http://www.ncbi.nlm.nih.gov/gorf/b12.html) by BLASTN (for nucleotide sequences) are reward for match =1, penalty for mismatch = -2, open gap = 5, extension gap = 2. When using BLASTP for protein sequences, the default parameters are reward for match = 0, penalty for mismatch = 0, open gap = 11, and extension gap =1.

When two sequences share "sequence homology," it is meant that the two sequences differ from each other only by conservative substitutions. For polypeptide sequences, such conservative substitutions consist of substitution of one amino acid at a given position in the sequence for another amino acid of the same class (e.g., amino acids that share characteristics of hydrophobicity, charge, pK or other conformational or chemical properties, e.g., valine for leucine, arginine for lysine), or by one or more non-conservative amino acid substitutions, deletions, or insertions, located at positions of the sequence that do not alter the conformation or folding of the polypeptide to the extent that the biological activity of the polypeptide is destroyed. Examples of "conservative substitutions" include substitution of one non-polar (hydrophobic) residue such as isoleucine, valine, leucine or methionine for one another; the substitution of one polar (hydrophilic) residue for another such as between arginine and lysine, between glutamine and asparagine, between threonine and serine; the substitution of one basic residue such as lysine, arginine or histidine for one another; or the substitution of one acidic residue, such as aspartic acid or glutamic acid for one another; or the use of a chemically derivatized residue in place of a non-derivatized residue; provided that the polypeptide displays the requisite biological activity. Two sequences which share sequence homology may called "sequence homologs."

The invention contemplates mutants of the proteins and peptides disclosed herein, where the mutation(s) do not substantially alter the activity of the protein or peptide, that is the mutations are effectively "silent" mutations.

Homology, for polypeptides, is typically measured using sequence analysis software (e.g., Sequence Analysis Software Package of the Genetics Computer Group, University of Wisconsin Biotechnology Center, 1710 University Avenue, Madison, WI 53705). Protein analysis- software matches similar sequences by assigning degrees of homology to various substitutions, deletions, and other modifications. Conservative substitutions typically include substitutions within the following groups: glycine, alanine; valine, isoleucine, leucine; aspartic acid, glutamic acid, asparagine, glutamine; serine, threonine; lysine, arginine; and phenylalanine, tyrosine.

Also encompassed by the present invention are chemical derivatives of Vascostatin. "Chemical derivative" refers to a subject polypeptide having one or more residues chemically derivatized by reaction of a functional side group. Such derivatized residues include for example, those molecules in which free amino groups have been derivatized to form amine hydrochlorides, p-toluene sulfonyl groups, carbobenzoxy groups, t-butyloxycarbonyl groups, chloroacetyl groups or formyl groups. Free carboxyl groups may be derivatized to form salts, methyl and ethyl esters or other types of esters or hydrazides. Free hydroxyl groups may be derivatized to form O-acyl or O-alkyl derivatives. The imidazole nitrogen of histidine may be derivatized to form N-imbenzylhistidine. Also included as chemical derivatives are those peptides which contain one or more naturally occurring amino acid derivatives of the twenty standard amino acids. For examples: 4-hydroxyproline may be substituted for proline; 5-hydroxylysine may be substitute for lysine; 3-methylhistidine may be substituted for histidine; homoserine may be substituted for serine; and ornithine may be substituted for lysine.

The present invention also includes fusion proteins and chimeric proteins comprising the anti-angiogenic proteins, their fragments, mutants, homologs, analogs, and allelic variants. A fusion or chimeric protein can be produced as a result of recombinant expression and the cloning process, *e.g.*, the protein may be produced comprising additional amino acids or amino acid sequences corresponding to full or partial linker sequences. A fusion or chimeric protein can consist of a multimer of a single protein, e.g., repeats of the anti-angiogenic proteins, or the fusion and chimeric proteins can be made up of several proteins, *e.g.*, several of the anti-angiogenic proteins. The fusion or chimeric protein can comprise a combination of two or more known anti-angiogenic proteins *(e.g.,* angiostatin and endostatin, or biologically active fragments of angiostatin and endostatin), or an anti-angiogenic protein in combination with a targeting agent (e.g., endostatin with epidermal growth factor (EGF) or RGD peptides), or an anti-angiogenic protein in combination with an immunoglobulin molecule *(e.g.,* endostatin and IgG, specifically with the Fc portion removed). The fusion and chimeric proteins can also include the anti-angiogenic proteins, their fragments, mutants, homologs, analogs, and allelic variants, and other anti-angiogenic proteins, e.g., endostatin, or angiostatin. Other anti-angiogenic proteins can include Arresten, Canstatin or Tumstatin (PCT/US99/13737), Matin, restin and apomigren (PCT/US98/26058) and fragments of endostatin (PCT/US98/26057). The term "fusion protein" or "chimeric protein" as used herein can also encompass additional components for *e.g.*, delivering a chemotherapeutic agent, wherein a polynucleotide encoding the chemotherapeutic agent is linked to the polynucleotide encoding the anti-angiogenic protein. Fusion or chimeric proteins can also encompass multimers of an anti-angiogenic protein, *e.g.,* a dimer or trimer. Such fusion or chimeric proteins can be linked together via post-translational modification (e.g., chemically linked), or the entire fusion protein may be made recombinantly.

Multimeric proteins comprising Vascostatin, its fragments, mutants, homologs, analogs and allelic variants are also intended to be encompassed by the present invention. By "multimer" is meant a protein comprising two or more copies of a subunit protein. The subunit protein may be one of the proteins of the present invention, *e.g.*, Vascostatin repeated two or more times, or a fragment, mutant, homolog, analog or allelic variant, *e.g.*, a Vascostatin mutant or fragment, repeated two or more times. Such a multimer may also be a fusion or chimeric protein, e.g., a repeated Tumstatin mutant may be combined with polylinker sequence, and/or one or more anti-angiogenic peptides, which may be present in a single copy, or may also be tandemly repeated, *e.g.,* a protein may comprise two or more multimers within the overall protein.

The invention also encompasses a composition comprising one or more isolated polynucleotide(s) encoding Vascostatin, as well as vectors and host cells containing such a polynucleotide, and processes for producing Vascostatin and its fragments, mutants, homologs, analogs and allelic variants. The term "vector" as used herein means a carrier into which pieces of nucleic acid may be inserted or cloned, which carrier functions to transfer the pieces of nucleic acid into a host cell. Such a vector may also bring about the replication and/or expression of the transferred nucleic acid pieces. Examples of vectors include nucleic acid molecules derived, e.g., from a plasmid, bacteriophage, or mammalian, plant or insect virus, or non-viral vectors such as ligand-nucleic acid conjugates, liposomes, or lipid-nucleic acid complexes. It may be desirable that the transferred nucleic molecule is operatively linked to an expression control sequence to form an expression vector capable of expressing the transferred nucleic acid Such transfer of nucleic acids is generally called "transformation," and refers to the insertion of an exogenous polynucleotide into a host cell, irrespective of the method used for the insertion. For example, direct uptake, transduction or f-mating are included. The exogenous polynucleotide may be maintained as a non-integrated vector, for example, a plasmid, or alternatively, may be integrated into the host genome. "Operably linked" refers to a situation wherein the components described are in a relationship permitting them to function in their intended manner, e.g., a control sequence "operably linked" to a coding sequence is ligated in such a manner that expression of the coding sequence is achieved under conditions compatible with the control sequence. A "coding sequence" is a polynucleotide sequence which is transcribed into mRNA and translated into a polypeptide when placed under the control of (*e.g.*, operably linked to) appropriate regulatory sequences. The boundaries of the coding sequence are determined by a translation start codon at the 5'-terminus and a translation stop codon at the 3'-terminus. Such boundaries can be naturally-occurring, or can be introduced into or added the polynucleotide sequence by methods known in the art. A coding sequence can include, but is not limited to, mRNA, cDNA, and recombinant polynucleotide sequences.

The vector into which the cloned polynucleotide is cloned may be chosen because it functions in a prokaryotic, or alternatively, it is chosen because it functions in a eukaryotic organism. Two examples of vectors which allow for both the cloning of a polynucleotide encoding the Vascostatin protein, and the expression of that protein from the polynucleotide, are the pET22b and pET28(a) vectors (Novagen, Madison, Wisconsin, USA) and a modified pPICZαA vector (InVitrogen, San Diego, California, USA), which allow expression of the protein in bacteria and yeast, respectively, (see for example, WO 99/29878 and U.S.SN. 09/589,483).

Once a polynucleotide has been cloned into a suitable vector, it can be transformed into an appropriate host cell. By "host cell" is meant a cell which has been or can be used as the recipient of transferred nucleic acid by means of a vector. Host cells can prokaryotic or eukaryotic, mammalian, plant, or insect, and can exist as single cells, or as a collection, e.g., as a culture, or in a tissue culture, or in a tissue or an organism. Host cells can also be derived from normal or diseased tissue from a multicellular organism, *e.g.,* a mammal. Host cell, as used herein, is intended to include not only the original cell which was transformed with a nucleic acid, but also descendants of such a cell, which still contain the nucleic acid.

In one embodiment, the isolated polynucleotide encoding the anti-angiogenic protein additionally comprises a polynucleotide linker encoding a peptide. Such linkers are known to those of skill in the art and, for example the linker can comprise at least one additional codon encoding at least one additional amino acid. Typically the linker comprises one to about twenty or thirty amino acids. The polynucleotide linker is translated, as is the polynucleotide encoding the anti-angiogenic protein, resulting in the expression of an anti-angiogenic protein with at least one additional amino acid residue at the amino or carboxyl terminus of the anti-angiogenic protein. Importantly, the additional amino acid, or amino acids, do not compromise the activity of the anti-angiogenic protein.

After inserting the selected polynucleotide into the vector, the vector is transformed into an appropriate prokaryotic strain and the strain is cultured (*e.g.*, maintained) under suitable culture conditions for the production of the biologically active anti-angiogenic protein, thereby producing a biologically active anti-angiogenic protein, or mutant, derivative, fragment or fusion protein thereof. In one embodiment, the invention comprises cloning of a polynucleotide encoding an anti-angiogenic protein into the vectors pET22b, pET17b or pET28a, which are then transformed into bacteria. The bacterial host strain then expresses the anti-angiogenic protein. Typically the anti-angiogenic proteins are produced in quantities of about 10-20 milligrams, or more, per liter of culture fluid.

In another embodiment of the present invention, the eukaryotic vector comprises a modified yeast vector. One method is to use a pPICzα plasmid wherein the plasmid contains a multiple cloning site. The multiple cloning site has inserted into the multiple cloning site a His.Tag motif. Additionally the vector can be modified to add a *Nde*I site, or other suitable restriction sites. Such sites are well known to those of skill in the art. Anti-angiogenic proteins produced by this embodiment comprise a histidine tag motif (His.tag) comprising one, or more histidines, typically about 5-20 histidines. The tag must not interfere with the anti-angiogenic properties of the protein.

One method of producing Vascostatin, for example, is to amplify the polynucleotide of SEQ ID NO:1 and clone it into an expression vector, e.g., pET22b, pET28(a), pPICZαA, or some other expression vector, transform the vector containing the polynucleotide into a host cell capable of expressing the polypeptide encoded by the polynucleotide, culturing the transformed host cell under culture conditions suitable for expressing the protein, and then extracting and purifying the protein from the culture. Exemplary methods of producing anti-angiogenic proteins in general, are provided in the Examples below and in U.S.S.N. 09/335, 224, "Anti-Angiogenic Proteins and Methods of Use Thereof," by Raghuram Kalluri, filed June 17, 1999. The Vascostatin protein may also be expressed as a product of transgenic animals, e.g., as a component of the milk of transgenic cows, goats, sheep or pigs, as is described in U.S. Pat. No. 5,962,648, or as a product of a transgenic plant, e.g., combined or linked with starch molecules in maize, or as is described in U.S. Pat. No. 5,639,947 or 5,990,385.

Vascostatin may also be produced by conventional, known methods of chemical synthesis. Methods for constructing the proteins of the present invention by synthetic means are known to those skilled in the art. The synthetically-constructed Vascostatin protein sequence, by virtue of sharing primary, secondary or tertiary structural and/or conformational characteristics with e.g., recombinantly-produced Vascostatin, may possess biological properties in common therewith, including biological activity. Thus, the synthetically-constructed Vascostatin protein sequence may be employed as biologically active or immunological substitutes for *e*.*g*., recombinantly-produced, purified Vascostatin protein in screening of therapeutic compounds and in immunological processes for the development of antibodies.

The Vascostatin protein is useful in inhibiting angiogenesis, as determined in standard assays, and provided in the Examples below.

Polynucleotides encoding Vascostatin can be cloned out of isolated DNA or a cDNA library. Nucleic acids polypeptides, referred to herein as "isolated" are nucleic acids or polypeptides substantially free (*i.e.*, separated away frorn) the material of the biological source from which they were obtained (*e.g.*, as exists in a mixture of nucleic acids or in cells), which may have undergone further processing. "Isolated" nucleic acids or polypeptides include nucleic acids or polypeptides obtained by methods described herein, similar methods, or other suitable methods, including essentially pure nucleic acids or polypeptides, nucleic acids or polypeptides produced by chemical synthesis, by combinations of chemical or biological methods, and recombinantly produced nucleic acids or polypeptides which are isolated. An isolated polypeptide therefore means one which is relatively free of other proteins, carbohydrates, lipids, and other cellular components with which it is normally associated. An isolated nucleic acid is not immediately contiguous with (*i.e.,* covalently linked to) both of the nucleic acids with which it is immediately contiguous in the naturally-occurring genome of the organism from which the nucleic acid is derived. The term, therefore, includes, for example, a nucleic acid which is incorporated into a vector (e.g., an autonomously replicating virus or plasmid), or a nucleic acid which exists as a separate molecule independent of other nucleic acids such as a nucleic acid fragment produced by chemical means or restriction endonuclease treatment.

The polynucleotides and proteins of the present invention can also be used to design probes to isolate other anti-angiogenic proteins. Exceptional methods are provided in U.S. Pat. No. 5,837,490, by Jacobs *et al.,*. The design of the oligonucleotide probe should preferably follow these parameters: (a) it should be designed to an area of the sequence which has the fewest ambiguous bases ("N's"), if any, and (b) it should be designed to have a Tₘ of approx. 80°C (assuming 2°C for each A or T and 4°C for each G or C).

The oligonucleotide should preferably be labeled with g-³²P-ATP (specific activity 6000 Ci/mmole) and T4 polynucleotide kinase using commonly employed techniques for labeling oligonucleotides. Other labeling techniques can also be used. Unincorporated label should preferably be removed by gel filtration chromatography or other established methods. The amount of radioactivity incorporated into the probe should be quantitated by measurement in a scintillation counter. Preferably, specific activity of the resulting probe should be approximately 4 × 10⁶ dpm/pmole. The bacterial culture containing the pool of full-length clones should preferably be thawed and 100 µl of the stock used to inoculate a sterile culture flask containing 25 ml of sterile L-broth containing ampicillin at 100 µg/ml. The culture should preferably be grown to saturation at 37°C, and the saturated culture should preferably be diluted in fresh L-broth. Aliquots of these dilutions should preferably be plated to determine the dilution and volume which will yield approximately 5000 distinct and well-separated colonies on solid bacteriological media containing L-broth containing ampicillin at 100 µg/ml and agar at 1.5% in a 150 mm petri dish when grown overnight at 37°C. Other known methods of obtaining distinct, well-separated colonies can also be employed.

Standard colony hybridization procedures should then be used to transfer the colonies to nitrocellulose filters and lyse, denature and bake them. Highly stringent condition are those that are at least as stringent as, for example, 1x SSC at 65°C, or 1x SSC and 50% formamide at 42°C. Moderate stringency conditions are those that are at least as stringent as 4x SSC at 65°C, or 4x SSC and 50% formamide at 42°C. Reduced stringency conditions are those that are at least as stringent as 4x SSC at 50°C, or 6x SSC and 50% formamide at 40°C.

The filter is then preferably incubated at 65°C for 1 hour with gentle agitation in 6x SSC (20x stock is 175.3 g NaCl/liter, 88.2 g Na citrate/liter, adjusted to pH 7.0 with NaOH) containing 0.5% SDS, 100 µg/ml of yeast RNA, and 10 mM EDTA (approximately 10 mL per 150 mm filter). Preferably, the probe is then added to the hybridization mix at a concentration greater than or equal to 1 x 10⁶ dpm/mL. The filter is then preferably incubated at 65°C with gentle agitation overnight. The filter is then preferably washed in 500 mL of 2x SSC/0.5% SDS at room temperature without agitation, preferably followed by 500 mL of 2x SSC/0.1% SDS at room temperature with gentle shaking for 15 minutes. A third wash with 0.1x SSC/0.5% SDS at 65°C for 30 minutes to 1 hour is optional. The filter is then preferably dried and subjected to autoradiography for sufficient time to visualize the positives on the X-ray film. Other known hybridization methods can also be employed. The positive colonies are then picked, grown in culture, and plasmid DNA isolated using standard procedures. The clones can then be verified by restriction analysis, hybridization analysis, or DNA sequencing.

Stringency conditions for hybridization refers to conditions of temperature and buffer composition which permit hybridization of a first nucleic acid sequence to a second nucleic acid sequence, wherein the conditions determine the degree of identity between those sequences which hybridize to each other. Therefore, "high stringency conditions" are those conditions wherein only nucleic acid sequences which are very similar to each other will hybridize. The sequences may be less similar to each other if they hybridize under moderate stringency conditions. Still less similarity is needed for two sequences to hybridize under low stringency conditions. By varying the hybridization conditions from a stringency level at which no hybridization occurs, to a level at which hybridization is first observed, conditions can be determined at which a given sequence will hybridize to those sequences that are most similar to it. The precise conditions determining the stringency of a particular hybridization include not only the ionic strength, temperature, and the concentration of destabilizing agents such as formamide, but also on factors such as the length of the nucleic acid sequences, their base composition, the percent of mismatched base pairs between the two sequences, and the frequency of occurrence of subsets of the sequences (e.g., small stretches of repeats) within other non-identical sequences. Washing is the step in which conditions are set so as to determine a minimum level of similarity between the sequences hybridizing with each other. Generally, from the lowest temperature at which only homologous hybridization occurs, a 1 % mismatch between two sequences results in a 1°C decrease in the melting temperature (Tₘ) for any chosen SSC concentration. Generally, a doubling of the concentration of SSC results in an increase in the Tₘ of about 17°C. Using these guidelines, the washing temperature can be determined empirically, depending on the level of mismatch sought. Hybridization and wash conditions are explained in *Current Protocols in Molecular-Biology* (Ausubel, *F.M. et al.,* eds., John Wiley & Sons, Inc., 1995, with supplemental updates) on pages 2.10.1 to 2.10.16, and 6.3.1 to 6.3.6.

High stringency conditions can employ hybridization at either (1) 1x SSC (10x SSC = 3 M NaCl, 0.3 M Na₃₋citrate·2H₂O (88 g/liter), pH to 7.0 with 1 M HCl), 1% SDS (sodium dodecyl sulfate), 0.1 - 2 mg/ml denatured salmon sperm DNA at 65°C, (2) 1x SSC, 50% formamide, 1% SDS, 0.1 - 2 mg/ml denatured salmon sperm DNA at 42°C, (3) 1% bovine serum albumen (fraction V), 1 mM Na₂-EDTA, 0.5 M NaHPO₄ (pH 7.2) (1 M NaHPO₄ = 134 g Na₂HPO₄·7H₂O, 4 ml 85% H₃PO₄ per liter), 7% SDS, 0.1 - 2 mg/ml denatured salmon sperm DNA at 65°C, (4) 50% formamide, 5x SSC, 0.02 M Tris-HCl (pH 7.6), 1x Denhardt's solution (100x =10 g Ficoll 400, 10 g polyvinylpyrrolidone, 10 g bovine serum albumin (fraction V), water to 500 ml), 10% dextran sulfate, 1% SDS, 0.1 - 2 mg/ml denatured salmon sperm DNA at 42°C, (5) 5x SSC, 5x Denhardt's solution, 1% SDS, 100 µg/ml denatured salmon sperm DNA at 65°C, or (6) 5x SSC, 5x Denhardt's solution, 50% formamide, 1% SDS, 100 µg/ml denatured salmon sperm DNA at 42°C, with high stringency washes of either (1) 0.3 - 0.1x SSC, 0.1% SDS at 65°C, or (2) 1 mM Na₂EDTA, 40 mM NaHPO₄ (pH 7.2), 1% SDS at 65°C. The above conditions are intended to be used for DNA-DNA hybrids of 50 base pairs or longer. Where the hybrid is believed to be less than 18 base pairs in length, the hybridization and wash temperatures should be 5 - 10°C below that of the calculated Tₘ of the hybrid, where Tₘ in °C = (2 x the number of A and T bases) + (4 x the number of G and C bases). For hybrids believed to be about 18 to about 49 base pairs in length, the Tₘ in °C = (81.5°C + 16.6(log₁₀M) + 0.41(% G + C) - 0.61 (% formamide) - 500/L), where "M" is the molarity of monovalent cations (e.g., Na⁺), and "L" is the length of the hybrid in base pairs.

Moderate stringency conditions can employ hybridization at either (1) 4x SSC, (10x SSC = 3 M NaCl, 0.3 M Na₃-citrate·2H₂O (88 g/liter), pH to 7.0 with 1 M HCl), 1% SDS (sodium dodecyl sulfate), 0.1 - 2 mg/ml denatured salmon sperm DNA at 65°C, (2) 4x SSC, 50% formamide, 1% SDS, 0.1 - 2 mg/ml denatured salmon sperm DNA at 42°C, (3) 1% bovine serum albumen (fraction V), 1 mM Na₂·EDTA, 0.5 M NaHPO₄ (pH 7.2) (1 M NaHPO₄ = 134 g Na₂HPO₄·7H₂O, 4 ml 85% H₃PO₄ per liter), 7% SDS, 0.1 - 2 mg/ml denatured salmon sperm DNA at 65°C, (4) 50% formamide, 5x SSC, 0.02 M Tris-HCl (pH 7.6), 1x Denhardt's solution (100x = 10 g Ficoll 400, 10 g polyvinylpyrrolidone, 10 g bovine serum albumin (fraction V), water to 500 ml), 10% dextran sulfate, 1% SDS, 0.1 - 2 mg/ml denatured salmon sperm DNA at 42°C, (5) 5x SSC, 5x Denhardt's solution, 1% SDS, 100 µg/ml denatured salmon sperm DNA at 65°C, or (6) 5x SSC, 5x Denhardt's solution, 50% formamide, 1% SDS, 100 µg/ml denatured salmon sperm DNA at 42°C, with moderate stringency washes of 1x SSC, 0.1% SDS at 65°C. The above conditions are intended to be used for DNA-DNA hybrids of 50 base pairs or longer. Where the hybrid is believed to be less than 18 base pairs in length, the hybridization and wash temperatures should be 5 - 10°C below that of the calculated Tₘ of the hybrid, where Tₘ in °C = (2 x the number of A and T bases) + (4 x the number of G and C bases). For hybrids believed to be about 18 to about 49 base pairs in length, the Tₘ in °C = (81.5°C + 16.6(log₁₀M) + 0.41(% G + C) - 0.61 (% formamide) - 500/L), where "M" is the molarity of monovalent cations (e.g., Na⁺), and "L" is the length of the hybrid in base pairs.

Low stringency conditions can employ hybridization at either (1) 4x SSC, (10x SSC = 3 M NaCl, 0.3 M Na₃-citrate·2H₂O (88 g/liter), pH to 7.0 with 1 M HCl), 1% SDS (sodium dodecyl sulfate), 0.1 - 2 mg/ml denatured salmon sperm DNA at 50°C, (2) 6x SSC, 50% formamide, 1% SDS, 0.1 - 2 mg/ml denatured salmon sperm DNA at 40°C, (3) 1% bovine serum albumen (fraction V), 1 mM Na₂-EDTA, 0.5 M NaHPO₄ (pH 7.2) (1 M NaHPO₄ = 134 g Na₂HPO₄·7H₂O, 4 ml 85% H₃PO₄ per liter), 7% SDS, 0.1 - 2 mg/ml denatured salmon sperm DNA at 50°C, (4) 50% formamide, 5x SSC, 0.02 M Tris-HCl (pH 7.6), 1x Denhardt's solution (100x = 10 g Ficoll 400, 10 g polyvinylpyrrolidone, 10 g bovine serum albumin (fraction V), water to 500 ml), 10% dextran sulfate, 1% SDS, 0.1 - 2 mg/ml denatured salmon sperm DNA at 40°C, (5) 5x SSC, 5x Denhardt's solution, 1% SDS, 100 [µg/ml denatured salmon sperm DNA at 50°C, or (6) 5x SSC, 5x Denhardt's solution, 50% formamide, 1% SDS, 100 µg/ml denatured salmon sperm DNA at 40°C, with low stringency washes of either 2x SSC, 0.1% SDS at 50°C, or (2) 0.5% bovine serum albumin (fraction V), 1 mM Na₂EDTA, 40 mM NaHPO₄ (pH 7.2), 5% SDS. The above conditions are intended to be used for DNA-DNA hybrids of 50 base pairs or longer. Where the hybrid is believed to be less than 18 base pairs in length, the hybridization and wash temperatures should be 5 - 10°C below that of the calculated Tₘ of the hybrid, where Tₘ in °C = (2 x the number of A and T bases) + (4 x the number of G and C bases). For hybrids believed to be about 18 to about 49 base pairs in length, the Tₘ in °C = (81.5°C + 16.6(log₁₀M) + 0.41 (% G + C) - 0.61 (% formamide) - 500/L), where "M" is the molarity of monovalent cations (e.g., Na⁺), and "L" is the length of the hybrid in base pairs.

Methods of inhibiting angiogenesis in mammalian tissue can use Vascostatin or its biologically-active fragments, analogs, homologs, derivatives or mutants. Methods of treating an angiogenesis-mediated disease can use an effective amount of one or more of the anti-angiogenic proteins, or one or more biologically active fragment thereof, or combinations of fragments that possess anti-angiogenic activity, or agonists and antagonists. An effective amount of anti-angiogenic protein is an amount sufficient to inhibit the angiogenesis which results in the disease or condition, thus completely, or partially, alleviating the disease or condition. Alleviation of the angiogenesis-mediated disease can be determined by observing an alleviation of symptoms of the disease, *e.g.*, a reduction in the size of a tumor, or arrested tumor growth. As used herein, the term "effective amount" also means the total amount of each active component of the composition or method that is sufficient to show a meaningful patient benefit, *i.e*., treatment, healing, prevention or amelioration of the relevant medical condition, or an increase in rate of treatment, healing, prevention or amelioration of such conditions. When applied to a combination, the term refers to combined amounts of the active ingredients that result in the therapeutic effect, whether administered in combination, serially or simultaneously. Angiogenesis-mediated diseases include, but are not limited to, cancers, solid tumors, blood-born tumors *(e.g.,* leukemias), tumor metastasis, benign tumors *(e.g.,* hemangiomas, acoustic neuromas, neurofibromas, organ fibrosis, trachomas, and pyogenic granulomas), rheumatoid arthritis, psoriasis, ocular angiogenic diseases *(e.g.,* diabetic retinopathy, retinopathy of prematurity, macular degeneration, corneal graft rejection, neovascular glaucoma, retrolental fibroplasia, rubeosis), Osler-Webber Syndrome, myocardial angiogenesis, plaque neovascularization, telangiectasia, hemophiliac joints, angiofibroma, and wound granulation. The anti-angiogenic proteins are useful in the treatment of diseases of excessive or abnormal stimulation of endothelial cells. These diseases include, but are not limited to, intestinal adhesions, Crohn's disease, atherosclerosis, scleroderma, fibrosis and hypertrophic scars (*i.e*., keloids). The anti-angiogenic proteins can be used as a birth control agent by preventing vascularization required for embryo implantation. The anti-angiogenic proteins are useful in the treatment of diseases that have angiogenesis as a pathologic consequence such as cat scratch disease *(Rochele minalia quintosa)* and ulcers *(Heliobacter pylori).* The anti-angiogenic proteins can also be used to prevent dialysis graft vascular access stenosis, and obesity, e.g., by inhibiting capillary formation in adipose tissue, thereby preventing its expansion. The anti-angiogenic proteins can also be used to treat localized (e.g., nonmetastisized) diseases. "Cancer" means neoplastic growth, hyperplastic or proliferative growth or a pathological state of abnormal cellular development and includes solid tumors, non-solid tumors, and any abnormal cellular proliferation, such as that seen in leukemia. As used herein, "cancer" also means angiogenesis-dependent cancers and tumors, *i.e.,* tumors that require for their growth (expansion in volume and/or mass) an increase in the number and density of the blood vessels supplying them with blood. "Regression" refers to the reduction of tumor mass and size as determined using methods well-known to those of skill in the art.

Alternatively, where an increase in angiogenesis is desired, e.g., in wound healing, or in post-infarct heart tissue, antibodies or antisera to the anti-angiogenic proteins can be used to block localized, native anti-angiogenic proteins and processes, and thereby increase formation of new blood vessels so as to inhibit atrophy of tissue.

The anti-angiogenic proteins may be used in combination with themselves, or other compositions and procedures for the treatment of diseases, e.g., Vascostatin and Matin can be combined in a pharmaceutical composition, one or more of their be combined in a composition, or a tumor may be treated by with surgery, radiation, chemotherapy, or immunotherapy, combined -angiogenic proteins and then the anti-angiogenic proteins may be - administered to the patient to extend the dormancy of micrometastases ze and inhibit the growth of any residual primary tumor. The anti-proteins, or fragments, antisera, receptor agonists, or receptor antagonists combinations thereof can also be combined with other anti-angiogenic or proteins, fragments, antisera, receptor agonists, receptor antagonists -angiogenic proteins (e.g., angiostatin, endostatin). Additionally, the ic proteins, or their fragments, antisera, receptor agonists, receptor ⊃r combinations thereof, are combined with pharmaceutically cipients, and optionally sustained-release matrix, such as biodegradable form therapeutic compositions. The compositions of the present ay also contain other anti-angiogenic proteins or chemical compounds, statin or angiostatin, and mutants, fragments, and analogs thereof. The may further contain other agents which either enhance the activity of compliment its activity or use in treatment, such as chemotherapeutic **e** agents. Such additional factors and/or agents may be included in the to produce a synergistic effect with protein of the invention, or to le effects. Additionally, administration of the composition of the tion may be administered concurrently with other therapies, e.g., - in conjunction with a chemotherapy or radiation therapy regimen. Angiongenenis can be inhibited in mannmalian tissues by contacting the tissue with a composition comprising the e invention. By "contacting" is meant not only topical application, but odes of delivery that introduce the composition into the tissues, or into ⊃e tissues. ⊃f timed release or sustained release delivery systems are also included ion. Such systems are highly desirable in situations where surgery is impossible, e.g., patients debilitated by age or the disease course itself, or k-benefit analysis dictates control over cure.

A sustained-release matrix, as used herein, is a matrix made of materials, usually polymers, which are degradable by enzymatic or acid/base hydrolysis or by dissolution. Once inserted into the body, the matrix is acted upon by enzymes and body fluids. The sustained-release matrix desirably is chosen from biocompatible materials such as liposomes, polylactides (polylactic acid), polyglycolide (polymer of glycolic acid), polylactide co-glycolide (co-polymers of lactic acid and glycolic acid) polyanhydrides, poly(ortho)esters, polyproteins, hyaluronic acid, collagen, chondroitin sulfate, carboxylic acids, fatty acids, phospholipids, polysaccharides, nucleic acids, polyamino acids, amino acids such as phenylalanine, tyrosine, isoleucine, polynucleotides, polyvinyl propylene, polyvinylpyrrolidone and silicone. A preferred biodegradable matrix is a matrix of one of either polylactide, polyglycolide, or polylactide co-glycolide (co-polymers of lactic acid and glycolic acid).

The angiogenesis-modulating composition of the present invention may be a solid, liquid or aerosol and may be administered by any known route of administration. Examples of solid compositions include pills, creams, and implantable dosage units. The pills may be administered orally, the therapeutic creams may be administered topically. The implantable dosage unit may be administered locally, for example at a tumor site, or which may be implanted for systemic release of the angiogenesis-modulating composition, for example subcutaneously. Examples of liquid composition include formulations adapted for injection subcutaneously, intravenously, intraarterially, and formulations for topical and intraocular administration. Examples of aerosol formulation include inhaler formulation for administration to the lungs.

The proteins and protein fragments with the anti-angiogenic activity described above can be provided as isolated and substantially purified proteins and protein fragments in pharmaceutically acceptable formulations using formulation methods known to those of ordinary skill in the art. These formulations can be administered by standard routes. In general, the combinations may be administered by the topical, transdermal, intraperitoneal, intracranial, intracerebroventricular, intracerebral, intravaginal, intrauterine, oral, rectal or parenteral (*e.g*., intravenous, intraspinal, subcutaneous or intramuscular) route. In addition, the anti-angiogenic proteins may be incorporated into biodegradable polymers allowing for sustained release of the compound, the polymers being implanted in the vicinity of where drug delivery is desired, for example, at the site of a tumor or implanted so that the anti-angiogenic proteins are slowly released systemically. Osmotic minipumps may also be used to provide controlled delivery of high concentrations of the anti-angiogenic proteins through cannulae to the site of interest, such as directly into a metastatic growth or into the vascular supply to that tumor. The biodegradable polymers and their use are described, for example, in detail in Brem *et al.* (1991, *J. Neurosurg.* 74:441-6).

The compositions containing a polypeptide of this invention can be administered intravenously, as by injection of a unit dose, for example. The term "unit dose" when used in reference to a therapeutic composition of the present invention refers to physically discrete units suitable as unitary dosage for the subject, each unit containing a predetermined quantity of active material calculated to produce the desired therapeutic effect in association with the required diluent; *i.e.,* carrier or vehicle.

Modes of administration of the compositions of the present inventions include intravenous, intramuscular, intraperitoneal, intrasternal, subcutaneous and intraarticular injection and infusion. Pharmaceutical compositions for parenteral injection comprise pharmaceutically acceptable sterile aqueous or nonaqueous, solutions, dispersions, suspensions or emulsions as well as sterile powders for reconstitution into sterile injectable solutions or dispersions just prior to use. Examples of suitable aqueous and nonaqueous carriers, diluents, solvents or vehicles include water, ethanol, polyois (*e.g.*, glycerol, propylene glycol, polyethylene glycol and the like), carboxymethylcellulose and suitable mixtures thereof, vegetable oils (e.g., olive oil) and injectable organic esters such as ethyl oleate. Proper fluidity may be maintained, for example, by the use of coating materials such as lecithin, by the maintenance of the required particle size in the case of dispersions and by the use of surfactants. These compositions may also contain adjuvants such as preservatives, wetting agents, emulsifying agents and dispersing agents. Prevention of the action of microorganisms may be ensured by the inclusion of various antibacterial and antifungal agents such as paraben, chlorobutanol, phenol sorbic acid and the like. It may also be desirable to include isotonic agents such as sugars, sodium chloride and the like. Prolonged absorption of the injectable pharmaceutical form may be brought about by the inclusion of agents, such as aluminum monostearate and gelatin, which delay absorption. Injectable depot forms are made by forming microencapsule matrices of the drug in biodegradable polymers such as polylactide-polyglycolide, poly(orthoesters) and poly(anhydrides). Depending upon the ratio of drug to polymer and the nature of the particular polmer employed, the rate of drug release can be controlled. Depot injectable formulations are also prepared by entrapping the drug in liposomes or microemulsions which are compatible with body tissues. The injectable formulations may be sterilized, for example, by filtration through a bacterial-retaining filter or by incorporating sterilizing agents in the form of sterile solid compositions which can be dissolved or dispersed in sterile water or other sterile injectable media just prior to use.

The therapeutic compositions of the present invention can include pharmaceutically acceptable salts of the components therein, e.g., which may be derived from inorganic or organic acids. By "pharmaceutically acceptable salt" is meant those salts which are, within the scope of sound medical judgement, suitable for use in contact with the tissues of humans and lower animals without undue toxicity, irritation, allergic response and the like and are commensurate with a reasonable benefit/risk ratio. Pharmaceutically acceptable salts are well-known in the art. For example, S. M. Berge, et al. describe pharmaceutically acceptable salts in detail in *J. Pharmaceutical Sciences* (1977) 66:1 *et seq.,.* Pharmaceutically acceptable salts include the acid addition salts (formed with the free amino groups of the polypeptide) that are formed with inorganic acids such as, for example, hydrochloric or phosphoric acids, or such organic acids as acetic, tartaric, mandelic and the like. Salts formed with the free carboxyl groups can also be derived from inorganic bases such as, for example, sodium, potassium, ammonium, calcium or ferric hydroxides, and such organic bases as isopropylamine, trimethylamine, 2-ethylamino ethanol, histidine, procaine and the like. The salts may be prepared *in situ* during the final isolation and purification of the compounds of the invention or separately by reacting a free base function with a suitable organic acid. Representative acid addition salts include, but are not limited to acetate, adipate, alginate, citrate, aspartate, benzoate, benzenesulfonate, bisulfate, butyrate, camphorate, camphorsufonate, digluconate, glycerophosphate, hemisulfate, heptonoate, hexanoate, fumarate, hydrochloride, hydrobromide, hydroiodide, 2-hydroxymethanesulfonate (isethionate), lactate, maleate, methanesulfonate, nicotinate, 2-naphthalenesulfonate, oxalate, pamoate, pectinate, persulfate, 3-phenylpropionate, picrate, pivalate, propionate, succinate, tartate, thiocyanate, phosphate, glutamate, bicarbonate, p-toluenesulfonate and undecanoate. Also, the basic nitrogen-containing groups can be quaternized with such agents as lower alkyl halides such as methyl, ethyl, propyl, and butyl chlorides, bromides and iodides; dialkyl sulfates like dimethyl, diethyl, dibutyl, and diamyl sulfates; long chain halides such as decyl, lauryl, myristyl and stearyl chlorides, bromides and iodides; arylalkyl halides like benzyl and phenethyl bromides and others. Water or oil-soluble or dispersible products are thereby obtained. Examples of acids which may be employed to form pharmaceutically acceptable acid addition salts include such inorganic acids as hydrochloric acid, hydrobromic acid, sulphuric acid and phosphoric acid and such organic acids as oxalic acid, maleic acid, succinic acid and citric acid.

As used herein, the terms "pharmaceutically acceptable," "physiologically tolerable" and grammatical variations thereof as they refer to compositions, carriers, diluents and reagents, are used interchangeably and represent that the materials are capable of administration to or upon a mammal with a minimum of undesirable physiological effects such as nausea, dizziness, gastric upset and the like. The preparation of a pharmacological composition that contains active ingredients dissolved or dispersed therein is well understood in the art and need not be limited based on formulation. Typically such compositions are prepared as injectables either as liquid solutions or suspensions, however, solid forms suitable for solution, or suspensions, in liquid prior to use can also be prepared. The preparation can also be emulsified.

The active ingredient can be mixed with excipients which are pharmaceutically acceptable and compatible with the active ingredient and in amounts suitable for use in the therapeutic methods described herein. Suitable excipients include, for example, water, saline, dextrose, glycerol, ethanol or the like and combinations thereof. In addition, if desired, the composition can contain minor amounts of auxiliary substances such as wetting or emulsifying agents, pH buffering agents and the like which enhance the effectiveness of the active ingredient.

The anti-angiogenic proteins of the present invention can also be included in a composition comprising a prodrug. As used herein, the term "prodrug" refers to compounds which are rapidly transformed *in vivo* to yield the parent compound, for example, by enzymatic hydrolysis in blood. A thorough discussion is provided in T. Higuchi and V. Stella, *Prodrugs as Novel Delivery Systems,* Vol. 14 of the ACS Symposium Series and in Edward B. Roche, ed., *Bioreversible Carriers in Drug Design,* American Pharmaceutical Association and Permagon Press, 1987. As used herein, the term "pharmaceutically acceptable prodrug" refers to (1) those prochugs of the compounds of the present invention which are, within the scope of sound medical judgement, suitable for use in contact with the tissues of humans and animals without undue toxicity, irritation, allergic response and the like, commensurate with a suitable benefit-to-risk ratio and effective for their intended use and (2) zwitterionic forms, where possible, of the parent compound.

The dosage of the anti-angiogenic proteins of the present invention will depend on the disease state or condition being treated and other clinical factors such as weight and condition of the human or animal and the route of administration of the compound. Depending upon the half-life of the anti-angiogenic proteins in the particular animal or human, the anti-angiogenic proteins can be administered between several times per day to once a week. It is to be understood that the present invention has application for both human and veterinary use. The methods of the present invention contemplate single as well as multiple administrations, given either simultaneously or over an extended period of time. In addition, the anti-angiogenic proteins can be administered in conjunction with other forms of therapy, e.g., chemotherapy, radiotherapy, or immunotherapy.

The anti-angiogenic protein formulations include those suitable for oral, rectal, ophthalmic (including intravitreal or intracameral), nasal, topical (including buccal and sublingual), intrauterine, vaginal or parenteral (including subcutaneous, intraperitoneal, intramuscular, intravenous, intradermal, intracranial, intratracheal, and epidural) administration. The anti-angiogenic protein formulations may conveniently be presented in unit dosage form and may be prepared by conventional pharmaceutical techniques. Such techniques include the step of bringing into association the active ingredient and the pharmaceutical carrier(s) or excipient(s). In general, the formulations are prepared by uniformly and intimately bringing into association the active ingredient with liquid carriers or finely divided solid carriers or both, and then, if necessary, shaping the product.

Formulations suitable for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose containers, for example, sealed ampules and vials, and may be stored in a freeze-dried (lyophilized) condition requiring only the addition of the sterile liquid carrier, for example, water for injections, immediately prior to use. Extemporaneous injection solutions and suspensions may be prepared from sterile powders, granules and tablets of the kind previously described.

When an effective amount of protein of the present invention is administered orally, the anti-angiogenic proteins of the present invention will be in the form of a tablet, capsule, powder, solution or elixir. When administered in tablet form, the pharmaceutical composition of the invention may additionally contain a solid carrier such as a gelatin or an adjuvant. The tablet, capsule, and powder contain from about 5 to 95% protein of the present invention, and preferably from about 25 to 90% protein of the present invention. When administered in liquid form, a liquid carrier such as water, petroleum, oils of animal or plant origin such as peanut oil, mineral oil, soybean oil, or sesame oil, or synthetic oils may be added. The liquid form of the pharmaceutical composition may further contain physiological saline solution, dextrose or other saccharide solution, or glycols such as ethylene glycol, propylene glycol or polyethylene glycol. When administered in liquid form, the pharmaceutical composition contains from about 0.5 to 90% by weight of protein of the present invention, and preferably from about 1 to 50% protein of the present invention.

When an effective amount of protein of the present invention is administered by intravenous, cutaneous or subcutaneous injection, protein of the present invention will be in the form of a pyrogen-free, parenterally acceptable aqueous solution. The preparation of such parenterally acceptable protein solutions, having due regard to pH, isotonicity, stability, and the like, is within the skill in the art. A preferred pharmaceutical composition for intravenous, cutaneous, or subcutaneous injection should contain, in addition to protein of the present invention, an isotonic vehicle such as Sodium Chloride Injection, Ringer's Injection, Dextrose Injection, Dextrose and Sodium Chloride Injection, Lactated Ringer's Injection, or other vehicle as known in the art. The pharmaceutical composition of the present invention may also contain stabilizers, preservatives, buffers, antioxidants, or other additives known to those of skill in the art.

The amount of protein of the present invention in the pharmaceutical composition of the present invention will depend upon the nature and severity of the condition being treated, and on the nature of prior treatments which the patient has undergone. Ultimately, the attending physician will decide the amount of protein of the present invention with which to treat each individual patient. Initially, the attending physician will administer low doses of protein of the present invention and observe the patient's response. Larger doses of protein of the present invention may be administered until the optimal therapeutic effect is obtained for the patient, and at that point the dosage is not increased further.

The duration of intravenous therapy using the pharmaceutical composition of the present invention will vary, depending on the severity of the disease being treated and the condition and potential idiosyncratic response of each individual patient. It is contemplated that the duration of each application of the protein of the present invention will be in the range of 12 to 24 hours of continuous intravenous administration. Ultimately the attending physician will decide on the appropriate duration of intravenous therapy using the pharmaceutical composition of the present invention.

Preferred unit dosage formulations are those containing a daily dose or unit, daily sub-dose, or an appropriate fraction thereof, of the administered ingredient. It should be understood that in addition to the ingredients, particularly mentioned above, the formulations of the present invention may include other agents conventional in the art having regard to the type of formulation in question. Optionally, cytotoxic agents may be incorporated or otherwise combined with the anti-angiogenic proteins, or biologically functional protein fragements thereof, to provide dual therapy to the patient.

The therapeutic compositions are also presently valuable for veterinary applications. Particularly domestic animals and thoroughbred horses, in addition to humans, are desired patients for such treatment with proteins of the present invention.

Cytotoxic agents such as ricin, can be linked to the anti-angiogenic proteins, and fragments thereof, thereby providing a tool for destruction of cells that bind the anti-angiogenic proteins. These cells may be found in many locations, including but not limited to, micrometastases and primary tumors. Proteins linked to cytotoxic agents are infused in a manner designed to maximize delivery to the desired location. For example, ricin-linked high affinity fragments are delivered through a cannula into vessels supplying the target site or directly into the target. Such agents are also delivered in a controlled manner through osmotic pumps coupled to infusion cannulae. A combination of antagonists to the anti-angiogenic proteins may be co-applied with stimulators of angiogenesis to increase vascularization of tissue. This therapeutic regimen provides an effective means of destroying metastatic cancer.

Additional treatment methods include administration of the anti-angiogenic proteins, fragments, analogs, antisera, or receptor agonists and antagonists thereof, linked to cytotoxic agents. It is to be understood that the anti-angiogenic proteins can be human or animal in origin. The anti-angiogenic proteins can also be produced synthetically by chemical reaction or by recombinant techniques in conjunction with expression systems. The anti-angiogenic proteins can also be produced by enzymatically cleaving isolated Nidogen to generate proteins having anti-angiogenic activity. The anti-angiogenic proteins may also be produced by compounds that mimic the action of endogenous enzymes that cleave Nidogen to the anti-angiogenic proteins. Production of the anti-angiogenic proteins may also be modulated by compounds that affect the activity of cleavage enzymes.

The present invention also encompasses gene therapy whereby a polynucleotide encoding the anti-angiogenic proteins, integrins, integrin subunits, or a mutant, fragment, or fusion protein thereof, is introduced and regulated in a patient. Various methods of transferring or delivering DNA to cells for expression of the gene product protein, otherwise referred to as gene therapy, are disclosed in *Gene Transfer into Mammalian Somatic Cells in vivo,* N. Yang (1992) *Crit. Rev. Biotechn*. 12(4):335-56, Gene therapy encompasses incorporation of DNA sequences into somatic cells or germ line cells for use in either ex vivo or in vivo therapy. Gene therapy functions to replace genes, augment normal or abnormal gene function, and to combat infectious diseases and other pathologies.

Strategies for treating these medical problems with gene therapy include therapeutic strategies such as identifying the defective gene and then adding a functional gene to either replace the function of the defective gene or to augment a slightly functional gene; or prophylactic strategies, such as adding a gene for the product protein that will treat the condition or that will make the tissue or organ more susceptible to a treatment regimen. As an example of a prophylactic strategy, a gene such as that encoding one or more of the anti-angiogenic proteins may be placed in a patient and thus prevent occurrence of angiogenesis; or a gene that makes tumor cells more susceptible to radiation could be inserted and then radiation of the tumor would cause increased killing of the tumor cells.

Many protocols for transfer of the DNA or regulatory sequences of the anti-angiogenic proteins are envisioned in this invention. Transfection of promoter sequences, other than one normally found specifically associated with the anti-angiogenic proteins, or other sequences which would increase production of the anti-angiogenic proteins are also envisioned as methods of gene therapy. An example of this technology is found in Transkaryotic Therapies, Inc., of Cambridge, Mass., using homologous recombination to insert a "genetic switch" that turns on an erythropoietin gene in cells. See *Genetic Engineering News,* Apr. 15, 1994. Such "genetic switches" could be used to activate the anti-angiogenic proteins (or their receptors) in cells not normally expressing those proteins (or receptors).

Gene transfer methods for gene therapy fall into three broad categories: physical (e.g., electroporation, direct gene transfer and particle bombardment), chemical (e.g., lipid-based carriers, or other non-viral vectors) and biological *(e.g.,* virus-derived vector and receptor uptake). For example, non-viral vectors may be used which include liposomes coated with DNA. Such liposome/DNA complexes may be directly injected intravenously into the patient. It is believed that the liposome/DNA complexes are concentrated in the liver where they deliver the DNA to macrophages and Kupffer cells. These cells are long lived and thus provide long term expression of the delivered DNA. Additionally, vectors or the "naked" DNA of the gene may be directly injected into the desired organ, tissue or tumor for targeted delivery of the therapeutic DNA.

Gene therapy methodologies can also be described by delivery site. Fundamental ways to deliver genes include *ex vivo* gene transfer, *in vivo* gene transfer, and *in vitro* gene transfer. In *ex vivo* gene transfer, cells are taken from the patient and grown in cell culture. The DNA is transfected into the cells, the transfected cells are expanded in number and then reimplanted in the patient. In *in vitro* gene transfer, the transformed cells are cells growing in culture, such as tissue culture cells, and not particular cells from a particular patient. These "laboratory cells" are transfected, the transfected cells are selected and expanded for either implantation into a patient or for other uses.

*In vivo* gene transfer involves introducing the DNA into the cells of the patient when the cells are within the patient. Methods include using virally mediated gene transfer using a noninfectious virus to deliver the gene in the patient or injecting naked DNA into a site in the patient and the DNA is taken up by a percentage of cells in which the gene product protein is expressed. Additionally, the other methods described herein, such as use of a "gene gun," may be used for in vitro insertion of the DNA or regulatory sequences controlling production of the anti-angiogenic proteins.

Chemical methods of gene therapy may involve a lipid based compound, not necessarily a liposome, to transfer the DNA across the cell membrane. Lipofectins or cytofectins, lipid-based positive ions that bind to negatively charged DNA, make a complex that can cross the cell membrane and provide the DNA into the interior of the cell. Another chemical method uses receptor-based endocytosis, which involves binding a specific ligand to a cell surface receptor and enveloping and transporting it across the cell membrane. The ligand binds to the DNA and the whole complex is transported into the cell. The ligand gene complex is injected into the blood stream and then target cells that have the receptor will specifically bind the ligand and transport the ligand-DNA complex into the cell.

Many gene therapy methodologies employ viral vectors to insert genes into cells. For example, altered retrovirus vectors have been used in *ex vivo* methods to introduce genes into peripheral and tumor-infiltrating lymphocytes, hepatocytes, epidermal cells, myocytes, or other somatic cells. These altered cells are then introduced into the patient to provide the gene product from the inserted DNA.

Viral vectors have also been used to insert genes into cells using in vivo protocols. To direct the tissue-specific expression of foreign genes, cis-acting regulatory elements or promoters that are known to be tissue-specific can be used. Alternatively, this can be achieved using *in situ* delivery of DNA or viral vectors to specific anatomical sites *in vivo.* For example, gene transfer to blood vessels *in vivo* was achieved by implanting *in vitro* transduced endothelial cells in chosen sites on arterial walls. The virus infected surrounding cells which also expressed the gene product. A viral vector can be delivered directly to the *in vivo* site, by a catheter for example, thus allowing only certain areas to be infected by the virus, and providing long-term, site specific gene expression. *In vivo* gene transfer using retrovirus vectors has also been demonstrated in mammary tissue and hepatic tissue by injection of the altered virus into blood vessels leading to the organs.

Viral vectors that have been used for gene therapy protocols include but are not limited to, retroviruses, other RNA viruses such as poliovirus or Sindbis virus, adenovirus, adeno-associated virus, herpes viruses, SV 40, vaccinia and other DNA viruses. Replication-defective murine retroviral vectors are the most widely utilized gene transfer vectors. Murine leukemia retroviruses are composed of a single strand RNA complexed with a nuclear core protein and polymerase (pol) enzymes, encased by a protein core (gag) and surrounded by a glycoprotein envelope (env) that determines host range. The genomic structure of retroviruses include the *gag, pol,* and *env* genes enclosed at by the 5' and 3' long terminal repeats (LTR). Retroviral vector systems exploit the fact that a minimal vector containing the 5' and 3' LTRs and the packaging signal are sufficient to allow vector packaging, infection and integration into target cells providing that the viral structural proteins are supplied in trans in the packaging cell line. Fundamental advantages of retroviral vectors for gene transfer include efficient infection and gene expression in most cell types, precise single copy vector integration into target cell chromosomal DNA, and ease of manipulation of the retroviral genome.

The adenovirus is composed of linear, double stranded DNA complexed with core proteins and surrounded with capsid proteins. Advances in molecular virology have led to the ability to exploit the biology of these organisms to create vectors capable of transducing novel genetic sequences into target cells *in vivo.* Adenoviral-based vectors will express gene product proteins at high levels. Adenoviral vectors have high efficiencies of infectivity, even with low titers of virus. Additionally, the virus is fully infective as a cell free virion so injection of producer cell lines is not necessary. Another potential advantage to adenoviral vectors is the ability to achieve long term expression of heterologous genes *in vivo.*

Mechanical methods of DNA delivery include fusogenic lipid vesicles such as liposomes or other vesicles for membrane fusion, lipid particles of DNA incorporating cationic lipid such as lipofectin, polylysine-mediated transfer of DNA, direct injection of DNA, such as microinjection of DNA into germ or somatic cells, pneumatically delivered DNA-coated particles, such as the gold particles used in a "gene gun," and inorganic chemical approaches such as calcium phosphate transfection. Particle-mediated gene transfer methods were first used in transforming plant tissue. With a particle bombardment device, or "gene gun," a motive force is generated to accelerate DNA-coated high density particles (such as gold or tungsten) to a high velocity that allows penetration of the target organs, tissues or cells. Particle bombardment can be used in *in vitro* systems, or with *ex vivo* or *in vivo* techniques to introduce DNA into cells, tissues or organs. Another method, ligand-mediated gene therapy, involves complexing the DNA with specific ligands to form ligand-DNA conjugates, to direct the DNA to a specific cell or tissue.

It has been found that injecting plasmid DNA into muscle cells yields high percentage of the cells which are transfected and have sustained expression of marker genes. The DNA of the plasmid may or may not integrate into the genome of the cells. Non-integration of the transfected DNA would allow the transfection and expression of gene product proteins in terminally differentiated, non-proliferative tissues for a prolonged period of time without fear of mutational insertions, deletions, or alterations in the cellular or mitochondrial genome. Long-term, but not necessarily permanent, transfer of therapeutic genes into specific cells may provide treatments for genetic diseases or for prophylactic use. The DNA could be reinjected periodically to maintain the gene product level without mutations occurring in the genomes of the recipient cells. Non-integration of exogenous DNAs may allow for the presence of several different exogenous DNA constructs within one cell with all of the constructs expressing various gene products.

Electroporation for gene transfer uses an electrical current to make cells or tissues susceptible to electroporation-mediated mediated gene transfer. A brief electric impulse with a given field strength is used to increase the permeability of a membrane in such a way that DNA molecules can penetrate into the cells. This technique can be used in *in vitro* systems, or with *ex vivo* or *in vivo* techniques to introduce DNA into cells, tissues or organs.

Carrier mediated gene transfer *in vivo* can be used to transfect foreign DNA into cells. The carrier-DNA complex can be conveniently introduced into body fluids or the bloodstream and then site-specifically directed to the target organ or tissue in the body. Both liposomes and polycations, such as polylysine, lipofectins or cytofectins, can be used. Liposomes can be developed which are cell specific or organ specific and thus the foreign DNA carried by the liposome will be taken up by target cells. Injection of immunoliposomes that are targeted to a specific receptor on certain cells can be used as a convenient method of inserting the DNA into the cells bearing the receptor. Another carrier system that has been used is the asialoglycoportein/polylysine conjugate system for carrying DNA to hepatocytes for *in vivo* gene transfer.

The transfected DNA may also be complexed with other kinds of carriers so that the DNA is carried to the recipient cell and then resides in the cytoplasm or in the nucleoplasm. DNA can be coupled to carrier nuclear proteins in specifically engineered vesicle complexes and carried directly into the nucleus.

Gene regulation of the anti-angiogenic proteins may be accomplished by administering compounds that bind to the gene encoding one of the anti-angiogenic proteins, or control regions associated with the gene, or its corresponding RNA transcript to modify the rate of transcription or translation. Additionally, cells transfected with a DNA sequence encoding the anti-angiogenic proteins may be administered to a patient to provide an *in vivo* source of those proteins. For example, cells may be transfected with a vector containing a nucleic acid sequence encoding the anti-angiogenic proteins. The transfected cells may be cells derived from the patient's normal tissue, the patient's diseased tissue, or may be non-patient cells.

For example, tumor cells removed from a patient can be transfected with a vector capable of expressing the proteins of the present invention, and re-introduced into the patient. The transfected tumor cells produce levels of the protein in the patient that inhibit the growth of the tumor. Patients may be human or non-human animals. Cells may also be transfected by non-vector, or physical or chemical methods known in the art such as electroporation, ionoporation, or via a "gene gun." Additionally, the DNA may be directly injected, without the aid of a carrier, into a patient. In particular, the DNA may be injected into skin, muscle or blood.

The gene therapy protocol for transfecting the anti-angiogenic proteins into a patient may either be through integration of the anti-angiogenic protein DNA into the genome of the cells, into minichromosomes or as a separate replicating or non-replicating DNA construct in the cytoplasm or nucleoplasm of the cell. Expression of the anti-angiogenic proteins may continue for a long-period of time or may be reinjected periodically to maintain a desired level of the protein(s) in the cell, the tissue or organ or a determined blood level.

Angiogenesis-inhibiting proteins of the present invention can be synthesized in a standard microchemical facility and purity checked with HPLC and mass spectrophotometry. Methods of protein synthesis, HPLC purification and mass spectrophotometry are commonly known to those skilled in these arts. The anti-angiogenic proteins are also produced in recombinant *E. coli* or yeast expression systems, and purified with column chromatography.

Different protein fragments of the intact anti-angiogenic proteins can be synthesized for use in several applications including, but not limited to the following; as antigens for the development of specific antisera, as agonists and antagonists active at binding sites of the anti-angiogenic proteins, as proteins to be linked to, or used in combination with, cytotoxic agents for targeted killing of cells that bind the anti-angiogenic proteins.

The synthetic protein fragments of the anti-angiogenic proteins have a variety of uses. The protein that binds to the receptor(s) of the anti-angiogenic proteins with high specificity and avidity is radiolabeled and employed for visualization and quantitation of binding sites using autoradiographic and membrane binding techniques. This application provides important diagnostic and research tools. Knowledge of the binding properties of the receptor(s) facilitates investigation of the transduction mechanisms linked to the receptor(s).

The anti-angiogenic proteins and proteins derived from them can be coupled to other molecules using standard methods. The amino and carboxyl termini of the anti-angiogenic proteins both contain tyrosine and lysine residues and are isotopically and nonisotopically labeled with many techniques, for example radiolabeling using conventional techniques (tyrosine residues-chloramine T, iodogen, lactoperoxidase; lysine residues-Bolton-Hunter reagent). These coupling techniques are well known to those skilled in the art. Alternatively, tyrosine or lysine is added to fragments that do not have these residues to facilitate labeling of reactive amino and hydroxyl groups on the protein. The coupling technique is chosen on the basis of the functional groups available on the amino acids including, but not limited to amino, sulfhydral, carboxyl, amide, phenol, and imidazole. Various reagents used to effect these couplings include among others, glutaraldehyde, diazotized benzidine, carbodiimide, and p-benzoquinone.

The anti-angiogenic proteins are chemically coupled to isotopes, enzymes, carrier proteins, cytotoxic agents, fluorescent molecules, chemiluminescent, bioluminescent and other compounds for a variety of applications. The efficiency of the coupling reaction is determined using different techniques appropriate for the specific reaction. For example, radiolabeling of a protein of the present invention with ¹²⁵I is accomplished using chloramine T and Na¹²⁵I of high specific activity. The reaction is terminated with sodium metabisulfite and the mixture is desalted on disposable columns. The labeled protein is eluted from the column and fractions are collected. Aliquots are removed from each fraction and radioactivity measured in a gamma counter. In this manner, the unreacted Na ¹²⁵I is separated from the labeled protein. The protein fractions with the highest specific radioactivity are stored for subsequent use such as analysis of the ability to bind to antisera of the anti-angiogenic proteins.

In addition, labeling the anti-angiogenic proteins with short lived isotopes enables visualization of receptor binding sites *in vivo* using positron emission tomography or other modem radiographic techniques to locate tumors with the proteins' binding sites.

Systematic substitution of amino acids within these synthesized proteins yields high affinity protein agonists and antagonists to the receptor(s) of the anti-angiogenic proteins that enhance or diminish binding to the receptor(s). Such agonists are used to suppress the growth of micrometastases, thereby limiting the spread of cancer. Antagonists to the anti-angiogenic proteins are applied in situations of inadequate vascularization, to block the inhibitory effects of the anti-angiogenic proteins and promote angiogenesis. For example, this treatment may have therapeutic effects to promote wound healing in diabetics.

The invention is further illustrated by the following examples, which are not meant to be construed in any way as imposing limitations upon the scope thereof.

### EXAMPLES

### Example 1: Recombinant Production of Vascostatin in E. coli.

Vascostatin is the C-terminal globular domain of nidogen-1 (Fig. 1). The nucleotide (SEQ ID NO:1) and amino acid (SEQ ID NO:2) sequences for Vascostatin (GenBank Acc. No. X14480) are shown in Figs. 2A and 2B. The sequence encoding Vascostatin was amplified by PCR using the forward primer 5'-CCC-AAG-CTT-AGA-GGC-ATT-GTG-ACA-GAC-3' (SEQ ID NO:3) and the reverse primer 5'-CCG-CTC-GAG-TTT-CCG-TTC-AAT-GCA-GTC-AAC-3' (SEQ ID NO:4). The resulting cDNA fragment was digested with *Hin*dIII and *Xho*I and ligated into predigested pET22b(+) (Novagen, Madison, Wisconsin, USA). The ligation placed Vascostatin in-frame with the pelB leader sequence, allowing for periplasmic localization and expression of soluble protein. The 3' end of the sequence was ligated in-frame with the polyhistidine tag sequence.

Plasmid constructs encoding Vascostatin were first transformed into *E. coli* HMS 174 (Novagen, Madison, Wisconsin, USA) and then transformed into BL21 for expression (Novagen, Madison, Wisconsin, USA). Overnight bacterial culture was used to inoculate a 500 ml culture in LB medium (Fisher Scientific, Pittsburgh, Pennsylvania, USA). This culture was grown for approximately 4 hours until the cells reached an OD₆₀₀ of 0.6. Protein expression was then induced by addition of IPTG to a final concentration of 1 mM. After a 2-hour induction, cells were harvested by centrifugation at 5,000 x g and lysed by resuspension in 6 M guanidine, 0.1 M NaH₂PO₄, 0.01 M Tris-HCl, pH 8.0. Resuspended cells were sonicated briefly, and centrifuged at 12,000 x g for 30 minutes. The supernatant fraction was passed over a 5 ml Ni-NTA agarose column (Qiagen, Hilden, Germany) 4-6 times at a speed of 2 ml per minute. Non-specifically bound protein was removed by washing with both 10 mM and 25 mM imidazole in 8 M urea, 0.1 M NaH₂PO₄, 0.01 M Tris-HCl, pH 8.0. Vascostatin protein was eluted from the column with increasing concentrations of imidazole (50 mM, 125 mM, and 250 mM) in 8 M urea, 0.1 M NaH₂PO₄, 0.01 M Tris-HCl, pH 8.0. The eluted protein was dialyzed twice against PBS at 4°C. A portion of the total protein precipitated during dialysis. Dialyzed protein was collected and centrifuged at approximately 3,500 x g and separated into insoluble (pellet) and soluble (supernatant) fractions.

E. coli-expressed Vascostatin was isolated predominantly as a soluble protein and SDS-PAGE analysis revealed a monomeric band at about 20 kDa. The eluted fractions containing this band were used in the following experiments. Protein concentration in each fraction was determined by the BCA assay (Pierce Chemical Co., Rockford, Illinois, USA) and quantitative SDS-PAGE analysis using scanning densitometry.

### Example 2: Vascostatin Inhibits Endothelial Cell Proliferation.

The anti-proliferative effect of Vascostatin on C-PAE cells was examined by the methylene blue staining assay using *E. coli* produced soluble protein.

*Cell lines and culture.* C-PAE (bovine pulmonary arterial endothelial cell line) cells were obtained from American Type Culture Collection. The C-PAE cell lines were maintained in DMEM (Life Technologies/Gibco BRL, Gaithersburg, Maryland, USA) supplemented with 10% fetal calf serum (FCS), 100 units/ml of penicillin, and 100 mg/ml of streptomycin.

*Proliferation assay.* C-PAE cells were either (1) grown to confluence in DMEM with 1% FBS and then stimulated with 10% FBS, with 1% FBS-stimulated cells serving as the control, (2) grown in 1% FBS and then stimulated with 10% FBS, with 0.1% FBS-stimulated cells serving as the control, or (3) kept contact-inhibited for 48 hours, then stimulated with 10% FBS, 10 ng/ml bFGF and 5 ng/ml VFGF.

Using the methylene-blue staining method, 7000 cells were plated into each well of a 96-well plate, and treated as described above. Cells were then counted using the method of Oliver *et al.* (Oliver, M.H. *et al., 1989, J. Cell. Sci.* 92:513-8).

The results are shown in Figs. 3A, 3B and 3C, which are three histograms showing the effect of Vascostatin on the proliferation of endothelial (C-PAE) cells. Absorbance at OD₆₅₅ is shown on the y-axis. The x-axis shows treatments varying amounts of FCS or Vascostatin. Fig. 3A shows treatments of 1% FCS, 10% FCS, and 0.01, 0.1, 1.0, 5.0,10.0 and 15.0 µg/ml Vascostatin, while Fig. 3B shows treatments of 0.1% FCS, 10% FCS, and 0.01, 0.1, 1.0, 5.0, 10.0 and 15.0 µg/ml Vascostatin, and Fig. 3C shows treatments of 10% FCS, and 0.001, 0.01, 0.1, 0.5, 1.0, 10.0, 15.0 and 20.0 µg/ml Vascostatin. Vascostatin inhibited the proliferation of endothelial cells in a dose-dependent manner.

## Claims

1. A protein of SEQ ID No:2, or a fragment of the protein comprising at least 25 continuous amino acids of said protein, or a variant of the protein having at least 70% sequence identity with SEQ ID No:2; wherein said protein, variant or fragment has anti-angiogenic activity.

2. A protein of Claim 1 that consists of the C-terminal globular domain of nidogen-1.

3. A variant according to Claim 1 having at least 80% sequence identity with said protein.

4. A variant according to Claim 1 having at least 90% sequence identity with said protein.

5. A protein, fragment, or variant according to any of Claims 1 to 4 that is a monomer.

6. A multimer of a protein, fragment, or variant according to any of Claims 1 to 4.

7. A chimeric protein comprising a protein, fragment or variant thereof according to any of Claims 1 to 4.

8. A chimeric protein according to Claim 7, further comprising at least one protein molecule selected from the group consisting of: matin or fragments thereof, arrestin or fragments thereof, canstatin or fragments thereof, tumstatin or fragments thereof, endostatin or fragments thereof, angiostatin or fragments thereof, restin or fragments thereof, apomigren or fragments thereof, or other anti-angiogenic proteins or fragments thereof.

9. A pharmaceutical or contraceptive composition comprising a protein, fragment, variant, monomer, multimer or chimeric protein according to any of Claims 1 to 8.

10. The composition of Claim 9, further comprising a pharmaceutically compatible carrier.

11. The composition of Claim 9 or 10, further comprising at least one protein molecule selected from the group consisting of: matin or fragments thereof, arrestin or fragments thereof, canstatin or fragments thereof, tumstatin or fragments thereof, endostatin or fragments thereof, angiostatin or fragments thereof, restin or fragments thereof, apomigren or fragments thereof, or other anti-angiogenic proteins, or fragments thereof.

12. A protein, fragment, variant, monomer, multimer, chimeric protein, or composition according to any of Claims 1 to 11; for use in medicine

13. The use of protein, fragment, variant, monomer, multimer, chimeric protein, or composition according to any of Claims 1 to 11 in the preparation of a medicament for treating a disorder involving angiogenesis.

14. The use according to Claim 13, wherein the disorder is cancer.

15. The use according to Claim 13, wherein the disorder involves tumor growth.

16. The use of a protein, fragment, variant, monomer, multimer, chimeric protein or composition according to any of Claims 1 to 11 in the preparation of a medicament for treating a disorder involving endothelial cell proliferation.

17. The use of a protein, fragment, variant, monomer, multimer, chimeric protein or composition according to any of Claims 1 to 11 in the preparation of a medicament for treating a disorder selected from the group comprising angiogenesis-dependent cancers, benign tumors, rheumatoid arthritis, diabetic retinopathy, fibrosis, psoriasis, ocular angiogenesis diseases, Osler-Webber Syndrome, myocardial angiogenesis, plaque neovascularization, telangiectasia, hemopheliac joints, angiofibroma, wound granulation, intestinal adhesions, atherosclerosis, scleroderma, hypertrophic scars, cat scratch disease, *Helicobacter pylori* ulcers, dialysis graft vascular access stenosis and obesity.

18. A combination of a protein, fragment, variant, monomer, multimer, chimeric protein or composition according to any of Claims 1 to 11 and a radiotherapeutic, chemotherapeutic or immunotherapeutic agent; for simultaneous, sequential or separate use in anti-cancer therapy.

19. A polynucleotide encoding the protein, fragment, variant or chimeric protein according to any of Claims 1 to 8.

20. The polynucleotide of Claim 19, wherein the polynucleotide is operably linked to an expression control sequence.

21. The polynucleotide according to Claim 19, wherein the polynucleotide has SEQ ID No 1.

22. The polynucleotide according to Claim 20, wherein the polynucleotide has 85% or greater sequence identity with SEQ ID No 1, or has 90% or greater sequence identity with SEQ ID No 1.

23. A host cell, e.g. a cell selected from the group comprising bacterial, yeast, mammalian, insect or plant cells, transformed with the polynucleotide of any of Claims 19 to 22; wherein said host cell is not part of a human embryo.

24. A polynucleotide according to any of Claims 19 to 22, or a host cell according to Claim 23; for use in medicine.

25. The use of a polynucleotide of any of Claims 19 to 22 in the preparation of a therapeutic mammalian cell for treating a mammal having a disorder as described in any of claims 13 to 17; wherein the preparation involves treating a mammalian cell *in vitro* to insert therein the polynucleotide; and wherein said cell is not part of a human embryo.

26. The use of Claim 25, wherein the cell allows transient expression.

27. The use of Claim 25 or 26, wherein the cell is chosen from the group consisting of: blood cells, TIL cells, bone marrow cells, vascular cells, tumor cells, liver cells, muscle cells, fibroblast cells.

28. The use of any of claims 25 to 27, wherein the polynucleotide is inserted into the cell by a viral vector.

29. A process for producing a protein, fragment, variant, monomer, multimer, or chimeric protein encoded by the polynucleotide of any of Claims 19 to 22, wherein the process comprises:
(a) growing a culture of a host cell according to claim 23; and
(b) purifying the protein, fragment, variant, monomer, multimer, or chimeric protein encoded by the polynucleotide from the culture.

30. A method comprising:
(a) preparing one or more polynucleotide probes that hybridize under conditions of moderate or high stringency to the polynucleotide of any of Claims 19 to 22;
(b) hybridizing said probe(s) to mammalian DNA; and
(c) isolating the DNA polynucleotide detected with the probe(s); wherein the nucleotide sequence of the isolated polynucleotide corresponds to the nucleotide sequence of the polynucleotide of any of Claims 19 to 22.

31. A protein, fragment, variant, monomer, multimer, or chimeric protein according to any of claims 1 to 8, a polynucleotide according to any of claims 19 to 22, or a host cell according to Claim 23; wherein the protein, fragment, variant, monomer, multimer, or chimeric protein is in isolated form.

## Patentansprüche

1. Ein Protein der SEQ ID NO: 2 oder ein Fragment des Proteins, das wenigstens 25 aufeinander folgende Aminosäuren des Proteins umfasst, oder eine Variante des Proteins mit wenigstens 70 % Sequenzidentität mit SEQ ID NO: 2, wobei das Protein, die Variante oder das Fragment anti-angiogene Aktivität aufweist.

2. Ein Protein von Anspruch 1, das aus der C-terminalen globulären Domäne von Nidogen-1 besteht.

3. Eine Variante gemäß Anspruch 1 mit wenigstens 80 % Sequenzidentität mit dem Protein.

4. Eine Variante gemäß Anspruch 1 mit wenigstens 90 % Sequenzidentität mit dem Protein.

5. Ein Protein, ein Fragment oder eine Variante gemäß einem der Ansprüche 1 bis 4, das (die) ein Monomer ist.

6. Ein Multimer eines Proteins, eines Fragments oder einer Variante gemäß einem der Ansprüche 1 bis 4.

7. Ein chimäres Protein, das ein Protein, ein Fragment oder eine Variante davon gemäß einem der Ansprüche 1 bis 4 umfasst.

8. Ein chimäres Protein gemäß Anspruch 7, das zusätzlich wenigstens ein Proteinmolekül umfasst, das aus der Gruppe ausgewählt ist, die aus: Matin oder Fragmenten davon, Arrestin oder Fragmenten davon, Canstatin oder Fragmenten davon, Tumstatin oder Fragmenten davon, Endostatin oder Fragmenten davon, Angiostatin oder Fragmenten davon, Restin oder Fragmenten davon, Apomigren oder Fragmenten davon oder anderen anti-angiogenen Proteinen oder Fragmenten davon besteht.

9. Eine pharmazeutische oder empfängnisverhütende Zusammensetzung, die ein Protein, ein Fragment, eine Variante, ein monomeres, multimeres oder chimäres Protein gemäß einem der Ansprüche 1 bis 8 umfasst.

10. Die Zusammensetzung von Anspruch 9, die zusätzlich ein pharmazeutisch verträgliches Trägermittel umfasst.

11. Die Zusammensetzung von Anspruch 9 oder 10, die zusätzlich wenigstens ein Proteinmolekül umfasst, das aus der Gruppe ausgewählt ist, die aus: Matin oder Fragmenten davon, Arrestin oder Fragmenten davon, Canstatin oder Fragmenten davon, Tumstatin oder Fragmenten davon, Endostatin oder Fragmenten davon, Angiostatin oder Fragmenten davon, Restin oder Fragmenten davon, Apomigren oder Fragmenten davon oder anderen anti-angiogenen Proteinen oder Fragmenten davon besteht.

12. Ein Protein, ein Fragment, eine Variante, ein monomeres, multimeres, chimäres Protein oder eine Zusammensetzung gemäß einem der Ansprüche 1 bis 11 zur Verwendung in einer Medizin.

13. Die Verwendung eines Proteins, eines Fragments, einer Variante, eines monomeren, multimeren oder chimären Proteins oder einer Zusammensetzung gemäß einem der Ansprüche 1 bis 11 zur Zubereitung eines Medikaments zur Behandlung einer Erkrankung, die Angiogenese involviert.

14. Die Verwendung gemäß Anspruch 13, wobei die Erkrankung Krebs ist.

15. Die Verwendung gemäß Anspruch 13, wobei die Erkrankung Tumorwachstum involviert.

16. Die Verwendung eines Proteins, eines Fragments, einer Variante, eines monomeren, multimeren, chimären Proteins oder einer Zusammensetzung gemäß einem der Ansprüche 1 bis 11 zur Zubereitung eines Medikaments zur Behandlung einer Erkrankung, die endotheliale Zellproliferation involviert.

17. Die Verwendung eines Proteins, eines Fragments, einer Variante, eines monomeren, multimeren, chimären Proteins oder einer Zusammensetzung gemäß einem der Ansprüche 1 bis 11 zur Zubereitung eines Medikaments zur Behandlung einer Erkrankung, die aus der Gruppe ausgewählt ist, die Angiogenese-abhängige Krebsarten, gutartige Tumore, rheumatoide Arthritis, diabetische Retinopathie, Fibrose, Psoriasis, okulare Angiogeneseerkrankungen, Osler-Webber-Syndrom, Herzmuskelangiogenese, Plaqueneovascularisierung, Telangiectasie, Blutergelenke, Angiofibrome, Wundgranulation, Darmverklebungen, Artheriosklerose, Scleroderma, hypertrophe Narben, Katzenkratzerkrankung, *Helicobacter pylori* Geschwüre, vaskuläre Dialysepfropfzugangsstenose und Fettleibigkeit umfasst.

18. Eine Kombination aus einem Protein, einem Fragment, einer Variante, einem monomeren, multimeren, chimären Protein oder einer Zusammensetzung gemäß einem der Ansprüche 1 bis 11 und einem radiotherapeutischen, chemotherapeutischen oder immuntherapeutischen Mittel zur gleichzeitigen, aufeinander folgenden oder getrennten Verwendung in einer Anti-Krebstherapie.

19. Ein Polynukleotid, das das Protein, das Fragment, die Variante oder das chimäre Protein gemäß einem der Ansprüche 1 bis 8 kodiert.

20. Das Polynukleotid von Anspruch 19, wobei das Polynukleotid operativ mit einer Expressionskontrollsequenz verbunden ist.

21. Das Polynukleotid gemäß Anspruch 19, wobei das Polynukleotid die SEQ ID NO: 1 aufweist.

22. Das Polynukleotid gemäß Anspruch 20, wobei das Polynukleotid eine 85 %-ige oder größere Sequenzidentität mit SEQ ID NO: 1 aufweist oder eine 90 %-ige oder größere Sequenzidentität mit SEQ ID NO: 1 aufweist.

23. Eine Wirtszelle, z. B. eine Zelle, die aus der Gruppe ausgewählt ist, die bakterielle, Hefe-, Säugetier-, Insekten- oder Pflanzenzellen umfasst, die mit dem Polynukleotid von einem der Ansprüche 19 bis 22 transformiert ist, wobei die Wirtszelle nicht Bestandteil eines menschlichen Embryos ist.

24. Ein Polynukleotid aus einem der Ansprüche 19 bis 22 oder eine Wirtszelle gemäß Anspruch 23 zur Verwendung in einer Medizin.

25. Die Verwendung eines Polynukleotids von einem der Ansprüche 19 bis 22 zur Zubereitung einer therapeutischen Säugetierzelle zur Behandlung eines Säugetiers mit einer Erkrankung wie sie in einem der Ansprüche 13 bis 17 beschrieben wird, wobei die Zubereitung die Behandlung einer Säugetierzelle *in vitro* zur Einführung des Polynukleotids darin involviert, und wobei die Zelle nicht Teil eines menschlichen Embryos ist.

26. Die Verwendung von Anspruch 25, wobei die Zelle die transiente Expression erlaubt.

27. Die Verwendung von Anspruch 25 oder 26, wobei die Zelle aus der Gruppe ausgewählt wird, die aus: Blutzellen, TIL-Zellen, Knochenmarkszellen, vaskulären Zellen, Tumorzellen, Leberzellen, Muskelzellen, und Fibroblastenzellen besteht.

28. Die Verwendung von einem der Ansprüche 25 bis 27, wobei das Polynukleotid in die Zelle durch eine viralen Vektor eingefügt wird.

29. Ein Verfahren zur Herstellung eines Proteins, eines Fragments, einer Variante, eines monomeren, multimeren oder chimären Proteins, das (die) durch das Polynukleotid von einem der Ansprüche 19 bis 22 kodiert wird, wobei das Verfahren das Folgende umfasst:
(a) das Wachsen einer Kultur aus einer Wirtszelle gemäß Anspruch 21, und
(b) das Aufreinigen des Proteins, des Fragments, der Variante, des monomeren, multimeren oder chimären Proteins aus der Kultur, das durch das Polynukleotid kodiert wird.

30. Ein Verfahren, umfassend:
(a) die Herstellung einer oder mehrerer Polynukleotidsonden, die unter Bedingungen moderater oder hoher Stringenz an das Polynukleotid von einem der Ansprüche 19 bis 22 hybridisieren,
(b) das Hybridisieren der Sonde(n) an Säugetier-DNA, und
(c) das Isolieren der mit der Sonde(n) nachgewiesenen DNA-Polynukleotids, wobei die Nukleotidsequenz des isolierten Polynukleotids mit der Nukleotidsequenz des Polynukleotids von einem der Ansprüche 19 bis 22 korrespondiert.

31. Ein Protein, ein Fragment, eine Variante, ein monomeres, multimeres oder chimäres Protein gemäß einem der Ansprüche 1 bis 8, ein Polynukleotid gemäß einem der Ansprüche 19 bis 22 oder eine Wirtszelle gemäß Anspruch 23, wobei das Protein, das Fragment, die Variante, das monomere, multimere oder chimäre Protein in isolierter Form vorliegt.

## Revendications

1. Protéine de SEQ ID NO:2, ou fragment de la protéine comprenant au moins 25 aminoacides continus de ladite protéine, ou variant de la protéine ayant au moins 70 % d'identité de séquence avec SEQ ID NO:2 ; où ladite protéine, ledit variant ou ledit fragment a une activité anti-angiogène.

2. Protéine selon la revendication 1 qui consiste en le domaine globulaire C-terminal de nidogène-1.

3. Variant selon la revendication 1 ayant au moins 80 % d'identité de séquence avec ladite protéine.

4. Variant selon la revendication 1 ayant au moins 90 % d'identité de séquence avec ladite protéine.

5. Protéine, fragment ou variant selon l'une quelconque des revendications 1 à 4 qui est un monomère.

6. Multimère d'une protéine, d'un fragment ou d'un variant selon l'une quelconque des revendications 1 à 4.

7. Protéine chimérique comprenant une protéine, un fragment ou un variant de celle-ci selon l'une quelconque des revendications 1 à 4.

8. Protéine chimérique selon la revendication 7 comprenant en outre au moins une molécule de protéine choisie dans le groupe consistant en : la matine ou des fragments de celle-ci, l'arrestine ou des fragments de celle-ci, la canstatine ou des fragments de celle-ci, la tumstatine ou des fragments de celle-ci, l'endostatine ou des fragments de celle-ci, l'angiostatine ou des fragments de celle-ci, la restine ou des fragments de celle-ci, l'apomigrène ou des fragments de celle-ci, ou d'autres protéines anti-angiogènes ou des fragments de celles-ci.

9. Composition pharmaceutique ou contraceptive comprenant une protéine, un fragment, un variant, un monomère, un multimère ou une protéine chimérique selon l'une quelconque des revendications 1 à 8.

10. Composition selon la revendication 9, comprenant en outre un support pharmaceutiquement compatible.

11. Composition selon la revendication 9 ou 10 comprenant en outre au moins une molécule de protéine choisie dans le groupe consistant en : la matine ou des fragments de celle-ci, l'arrestine ou des fragments de celle-ci, la canstatine ou des fragments de celle-ci, la tumstatine ou des fragments de celle-ci, l'endostatine ou des fragments de celle-ci, l'angiostatine ou des fragments de celle-ci, la restine ou des fragments de celle-ci, l'apomigrène ou des fragments de celle-ci, ou d'autres protéines anti-angiogène, ou des fragments de celles-ci.

12. Protéine, fragment, variant, monomère, multimère, protéine chimérique ou composition selon l'une quelconque des revendications 1 à 11 destinée à être utilisée en médecine.

13. Utilisation d'une protéine d'un fragment, d'un variant, d'un monomère, d'un multimère, d'une protéine chimérique ou d'une composition selon l'une quelconque des revendications 1 à 11 dans la préparation d'un médicament pour traiter une affection impliquant l'angiogenèse.

14. Utilisation selon la revendication 13 où l'affection est un cancer.

15. Utilisation selon la revendication 13 où l'affection implique la croissance tumorale.

16. Utilisation d'une protéine, d'un fragment, d'un variant, d'un monomère, d'un multimère, d'une protéine chimérique ou d'une composition selon l'une quelconque des revendications 1 à 11 dans la préparation d'un médicament pour traiter une affection impliquant la prolifération de cellules endothéliales.

17. Utilisation d'une protéine, d'un fragment, d'un variant, d'un monomère, d'un multimère, d'une protéine chimérique ou d'une composition selon l'une quelconque des revendications 1 à 11 dans la préparation d'un médicament pour traiter une affection choisie dans le groupe comprenant les cancers dépendants de l'angiogenèse, les tumeurs bénignes, la polyarthrite rhumatoïde, la rétinopathie diabétique, la fibrose, le psoriasis, les maladies d'angiogenèse oculaires, le syndrome de Osler-Webber, l'angiogenèse myocardique, la néovascularisation en plaques, les télangiectasies, l'arthropathie hémophilique, l'angiofibrome, la granulation des plaies, les adhésions intestinales, l'athérosclérose, la sclérodermie, les cicatrices hypertrophiques, la maladie des griffes de chat, les ulcères à *Helicobacter pylori,* la sténose d'accès vasculaire de greffe et de dialyse et l'obésité.

18. Combinaison d'une protéine, d'un fragment, d'un variant, d'un monomère, d'un multimère, d'une protéine chimérique ou d'une composition selon l'une quelconque des revendications 1 à 11 et d'un agent radiothérapeutique, chimiothérapeutique ou immunothérapeutique pour l'utilisation simultanée, successive ou séparée dans la thérapie anticancéreuse.

19. Polynucléotide codant la protéine, le fragment, le variant ou la protéine chimérique selon l'une quelconque des revendications 1 à 8.

20. Polynucléotide selon la revendication 19 où le polynucléotide est lié de manière fonctionnelle à une séquence de contrôle de l'expression.

21. Polynucléotide selon la revendication 19, où le polynucléotide a SEQ ID NO:1.

22. Polynucléotide selon la revendication 20, où le polynucléotide a 85 % d'identité de séquence ou plus avec SEQ ID NO:1, ou a 90 % d'identité de séquence ou plus avec SEQ ID NO:1.

23. Cellule hôte, par exemple cellule choisie dans le groupe comprenant les cellules bactériennes, de levure, de mammifères, d'insectes ou de plantes, transformée avec le polynucléotide selon l'une quelconque des revendications 19 à 22, où ladite cellule hôte ne fait pas partie d'un embryon humain.

24. Polynucléotide selon l'une quelconque des revendications 19 à 22, ou cellule hôte selon la revendication 23, destiné à être utilisé en médecine.

25. Utilisation d'un polynucléotide selon l'une quelconque des revendications 19 à 22 dans la préparation d'une cellule de mammifère thérapeutique pour traiter un mammifère ayant une affection telle que décrite dans l'une quelconque des revendications 13 à 17, où la préparation comprend le traitement d'une cellule de mammifère *in vitro* pour y insérer le polynucléotide, et où ladite cellule ne fait pas partie d'un embryon humain.

26. Utilisation selon la revendication 25, où la cellule permet une expression transitoire.

27. Utilisation selon la revendication 25 ou 26, où la cellule est choisie dans le groupe consistant en : les cellules sanguines, les cellules TIL, les cellules de moelle osseuse, les cellules vasculaires, les cellules tumorales, les cellules hépatites, les cellules musculaires, les cellules de fibroblastes.

28. Utilisation selon l'une quelconque des revendications 25 à 27, où le polynucléotide est inséré dans la cellule par un vecteur viral.

29. Procédé pour produire une protéine, un fragment, un variant, un monomère, un multimère ou une protéine chimérique codée par le polynucléotide selon l'une quelconque des revendications 19 à 22, où le procédé comprend :
(a) la croissance d'une culture d'une cellule hôte selon la revendication 23 ; et
(b) la purification de la protéine, du fragment, du variant, du monomère, du multimère ou de la protéine chimérique codée par le polynucléotide à partir de la culture.

30. Procédé comprenant :
(a) la préparation d'une ou plusieurs sondes polynucléotidiques qui s'hybrident dans des conditions de stringence modérée ou élevée au polynucléotide selon l'une quelconque des revendications 19 à 22 ;
(b) l'hybridation de ladite sonde ou desdites sondes à de l'ADN de mammifère ; et
(c) l'isolement du polynucléotide d'ADN détecté avec la sonde ou les sondes, où la séquence nucléotidique du polynucléotide isolé correspond à la séquence nucléotidique du polynucléotide selon l'une quelconque des revendications 19 à 22.

31. Protéine, fragment, variant, monomère, multimère ou protéine chimérique selon l'une quelconque des revendications 1 à 8, polynucléotide selon l'une quelconque des revendications 19 à 22, où cellule hôte selon la revendication 23, où la protéine, le fragment, le variant, le monomère, le multimère ou la protéine chimérique est sous forme isolée.
